# EUROPEAN PATENT APPLICATION

(11) **EP 4 512 395 A1**
(43) Date of publication of application: **26.02.2025**
(21) Application number: 23315322.0
(22) Date of filing: 21.08.2023
(51) Int. Cl.: A61K 31/20, A61K 31/513, A61K 31/7034, A61K 31/7076, A61K 31/7084, A61K 38/06, A61P 35/00, A61P 39/00

(54) **COMPOSITION COMPRISING FLAVIN ADENINE DINUCLEOTIDE (FAD), L-GSH, ATP AND MYRISTIC ACID, ALONE OR WITH A DRUG**

(71) Applicant: Bio Even, 75015 Paris (FR)
(72) Inventor: Arib, Célia, 72000 LE MANS (FR); Paleni, Didier, 25700 MATHAY (FR); Balzanelli, Mario Giosué, 74121 TARANTO (IT)
(74) Representative: INNOV-GROUP

(57) **Abstract**

The invention concerns a pharmaceutical or a nutraceutical composition comprising a salt of Flavine adenine dinucleotide (FAD), combined to ATP, or a salt thereof, reduced L-GSH and optionally myristic acid, or a salt thereof for use as a treatment against a disease and/or as a complement to reduce side effects induced by a drug, in particular an anti-cancer drug.

## Description

### FIELD OF THE INVENTION

The field of the invention generally relates to the field of pharmacy and/or nutraceutics.

### BACKGROUND

"A medicament" is a general term for a compound or a combination of compounds that can at least partially prevent or treat a disease when given to a patient. Depending on the dose used and on the patient's sensitivity, the medicament can have undesirable side effects and even induces death of the patient.

Thus, for example anti-cancer drugs that are used more and more frequently due to an increased incidence of cancers are those known to trigger very disabling side effects that affect patients' compliance to the treatment.

"Cancer" is a general term for a disease in which certain cells in the human body divide in an uncontrolled manner and are considered to have lost their ability to differentiate. These cells all derive from the same clone, cancer-initiating cell that has acquired particular characteristics enabling it to divide indefinitely. As the disease evolves, some cells may migrate from their place of production and form metastases. Thus, the resulting new cells may form a malignant tumor (a neoplasm) and/or spread throughout the body. Aging of the population and the improvement in diagnostics show an increase in the incidence of cancer. Cancer has thus become a public health issue.

To be effective a treatment must be effective in destroying cancerous cells while preserving the body's healthy cells as much as possible. However, the majority of treatments currently available are increasingly effective by being very aggressive towards cancer cells, but also towards healthy cells leading to undesirable effects that can be particularly disabling.

Thus, there is still a need in developing new treatments, in particular treatments targeting cancers with very poor prognosis or metastatic cancers.

There is also a need for a solution to palliate the undesired side effects induced by medications or drugs that induce very disabling sides effects, in particular anti-cancer drugs.

Liver cancer is the fifth most common cancer in the world and the third most common cause of cancer-related deaths. Llovet et al., 2003, "Hepatocellular carcinoma," Lancet 362: 1907-17. The most common form of liver cancer is hepatocellular carcinoma, also known as HCC, which often develops in connection with other basic liver damage, typically hepatitis or cirrhosis. Thorgeirsson et al., 2002, "Molecular pathogenesis of human hepatocellular carcinoma," Nature Genetics 31: 339-346. Less commonly, other forms of liver cancer include hepatoblastoma and bile duct cancer.

The standard treatment for liver cancer is surgical resection whenever possible, or chemotherapy, chemoembolization, or radiation therapy when there is no surgical option. Surgery is not always an option due to the size or location of the tumor and the reasons for progressive cirrhosis, and even if operated, tumor recurrence / re-occurrence exacerbates 70% of cases 5 years after resection. See Llovet et al (supra). Chemotherapy treatment also appears to be less effective in liver cancer, possibly due to the increased ability of hepatoma cells to excrete chemotherapeutic agents. There is therefore a significant and urgent need for a more effective treatment of liver cancer.

Similarly, cancer of the pancreas is the sixth most common cause of death due to cancer in France (7181 deaths in 2000), and the fifth most common cause of death due to cancer in the industrialized countries, and in the United States it has become the fourth most common cause of death in man. In France its incidence is from 5 to 10/100,000 inhabitants per year and is increasing slightly (1 to 2%) every year. It represents 7% of the cancers of the digestive system and affects men (60%) more often than women (40%). Since the diagnosis is often made at the advanced stage, metastases are detected in half of the cases, with the result that the average survival time in this cancer is only a few months with 4 to 6% of survivors at 5 years. The survival median, all stages combined, is from 4 to 7 months and it increases by 15 to 18 months in patients who have undergone resection.

If the tumor of the pancreas is not operable or exhibits metastases, chemotherapy may be considered in informed patients in good general condition. The response rates are of the order of 15 to 30%. The medicaments used are gemcitabine (Burris et al., European Journal of Cancer, 1997, 33:18-22), the combination gemcitabine and oxaliplatin (Zhao et al., Chinese-German Journal of Clinical Oncology, 2007, 6(5):461-463), and 5-fluorouracil (5-FU) in combination with a platinum derivative. These chemotherapies make it possible to moderately increase the survival median of patients with metastases, which is 4 to 6 months without treatment and have strong sides effects in particular weight loss.

In spite of the progress recorded with novel chemotherapies, the prognosis for cancer of the pancreas remains very poor. Even in patients operated for curative purposes, the survival rate is only about 20% at 5 years, owing to local and metastatic relapses.

In the face of the severity and very poor prognosis associated with cancer of the pancreas, and the progressive increase in its incidence in the population of Western countries in particular, there is a real need to propose an alternative treatment (s) more effective than those currently proposed.

Document WO2020089310A1 discloses a composition comprising FAD alone for the treatment of cancer.

### SUMMARY OF THE INVENTION

One purpose of the present invention is to provide a new anti-cancer treatment that is effective on cancer cells, in particular against aggressive cancers (progressing fast and/or with no or few treatments) such as pancreatic cancer or liver cancer cells, acting on an initial tumor or on cells spreading as a metastatic cancer while being as non-toxic as possible for healthy tissues.

Another objective is to provide an anti-cancer treatment combined with the composition of the invention to solve the problem of toxicity or side effect related said anti-cancer drug.

In addition, the researchers have discovered that the new composition of the invention can palliate the undesirable side effects of drugs or medications, and eventually increase not only compliance to the treatment by said drugs, better being during treatment but also survival to the disease whether the composition of the invention was given alone or in combination with another treatment.In some embodiments, the invention provides a composition comprising Flavine adenine dinucleotide (FAD) or a salt thereof, reduced glutathione (GSH), adenosine triphosphate (ATP) or a salt thereof, optionally myristic acid, or a salt thereof, and a carrier.ln some embodiments, the invention provides a composition according the above embodiment wherein the salt of FAD is Flavin adenine dinucleotide disodium salt hydrate, the salt of ATP is Adenosine Triphosphate disodium salt hydrate, GSH is reduced L-Glutathione.

In some embodiments, the invention provides a composition according to the above embodiments, wherein the carrier, is chosen from a metal salt, a polymer, a liposome, an excipient, a combination thereof.

In some embodiments, the invention provides a composition according to according the above embodiments, wherein the carrier comprises a polymer selected from polyethylene glycol (PEG), chitosan, collagen, starch, hyaluronic acid preferably a PEG, or chitosan or hyaluronic acid more preferably PEG 600; even more preferably PEG-600-diacid and even more more preferably hyaluronic acid.

In some embodiments, the invention provides a composition according the above embodiment, wherein the carrier comprises a metal salt, preferably a gold salt, more preferably HAuCl4*6H20.

In some embodiments, the invention provides a composition according the above embodiments in a form suitable for intravenous administration (iv), oral including sublingual administration (po), rectal administration, intratumoral administration, intraocular administration, intramucosal administration, intra-vesical administration, or intra-urethral administration, advantageously iv or po administration.

In some embodiments, the invention provides a composition according the above embodiments wherein the composition is administered to an individual at a dose ranging from 0,1mg to 10g for FAD or a salt thereof, a dose ranging from 0,1 to 10 g for GSH, a dose ranging from 0,1mg to 10g for ATP or a salt thereof and a dose ranging from 0.1mg to 10g for myristic acid or a salt thereof.

In some embodiments, the invention provides a composition according to the above embodiments in a therapeutically effective amount and a pharmaceutically acceptable carrier, or a nutraceutically acceptable carrier.

In some embodiments, the invention provides a composition, according to the above embodiments and a medicament.

In some embodiments, the invention provides a composition according to the above embodiments, for use in the prevention and/or the treatment of a disease.

In some embodiments, the invention provides a composition for its use according to the above embodiment wherein the disease is a cancer.

In some embodiments, the invention provides a composition according to the above embodiment for its use as an adjuvant (complement) or neoadjuvant (debulking) to cancer treatment.

in some embodiments, the invention provides a composition according the above embodiments wherein the disease is selected from the group consisting of brain cancer, breast cancer, prostate cancer, lung cancer, airway cancer, upper and/or lower digestive tract cancer, stomach cancer, kidney cancer, pancreas cancer, liver cancer, urinary tract cancer, genital organs cancer, skin cancer, Ear, Nose and Throat (ENT) sphere cancer, and lymphatic organs cancer, preferably pancreas cancer or liver cancer, more preferably aggressive of metastatic tumor cancer.
The composition according to the above embodiments for use in the prevention and/or the treatment of a side effect induced by a drug or a disease.

In some embodiments, the invention provides a composition according to the above embodiments for use in the prevention and/or the treatment of a side effect induced by 5-FU.

In some embodiments, the invention provides a composition according to the above embodiment for use in the prevention and/or the treatment of a side effect said side effect being selected from the list consisting of weight loss, nausea, vomiting, headache, bleeding, hair loss, neutropenia, pain, blood clots, lymphedema, a combination of said side effects, advantageously weight loss.

The invention provides as a main object a composition comprising Flavine adenine dinucleotide (FAD) or a salt thereof, reduced glutathione (GSH), or a salt thereof, adenosine triphosphate (ATP) or a salt thereof, optionally myristic acid, or a salt thereof, and a carrier. More particularly the invention relates to said composition for its uses, as such or in combination with another medicament potentially exhibiting side effects. The composition of the invention is provided wherein the salt of FAD is Flavine adenine dinucleotide disodium salt hydrate, the salt of ATP is Adenosine Triphosphate disodium salt hydrate, GSH is reduced L-Glutathione.

The composition of the invention is provided wherein the carrier, is chosen from a metal salt, a polymer, a liposome, an excipient, a combination thereof.

The composition of the invention is provided wherein the carrier comprises a polymer selected from polyethylene glycol (PEG), chitosan, collagen, starch, hyaluronic acid preferably a PEG, or chitosan or hyaluronic acid more preferably PEG 600; even more preferably PEG-600-diacid and even more more preferably hyaluronic acid.

The composition of the invention is provided wherein the carrier comprises a metal salt, preferably a gold salt, more preferably HAuCl₄*6H₂0.

The composition of the invention is provided in a form suitable for intravenous administration (iv), oral including sublingual administration (po), rectal administration, intratumoral administration, intraocular administration, intramucosal administration, intra-vesical administration, or intra-urethral administration, advantageously iv or po administration.

The composition of the invention is provided for administration to an individual at a dose ranging from 0,1mg to 10g for FAD or a salt thereof, at a dose ranging from 0,1 to 10 g for GSH, a dose ranging from 0,1mg to 10g for ATP or a salt thereof and a dose ranging from 0.1mg to 10g for myristic acid or a salt thereof.

The composition of the invention is provided in a therapeutically effective amount and a pharmaceutically acceptable carrier, or a nutraceutically acceptable carrier.
The composition of the invention is provided and a medicament.

The composition of the invention is provided for use in the prevention and/or the treatment of a disease.

The composition of the invention is provided for use in the prevention and/or the treatment of a cancer.

The composition of the invention is provided for its use as an adjuvant (complement) or neoadjuvant (debulking) to cancer treatment.

The composition of the invention is provided for use in the prevention and/or the treatment of a disease wherein the disease is selected from the group consisting of brain cancer, breast cancer, prostate cancer, lung cancer, airway cancer, upper and/or lower digestive tract cancer, stomach cancer, kidney cancer, pancreas cancer, liver cancer, urinary tract cancer, genital organs cancer, skin cancer, Ear, Nose and Throat (ENT) sphere cancer, and lymphatic organs cancer, preferably pancreas cancer or liver cancer, more preferably aggressive of metastatic tumor cancer.

The composition of the invention is provided for use in the prevention and/or the treatment of a side effect induced by a drug or a disease.
The composition of the invention is provided for use in the prevention and/or the treatment of a side effect induced by 5-FU.

The composition of the invention is provided for use in the prevention and/or the treatment of a side effect said side effect being selected from the list consisting of weight loss, nausea, vomiting, headache, bleeding, hair loss, neutropenia, pain, blood clots, lymphedema, a combination of said side effects, advantageously weight loss.

### Other embodiments encompass :

A composition comprising Flavin Adenine Dinucleotide, (FAD) or a salt thereof, and a vector characterized in that the composition further comprised adenosine triphosphate (ATP) or a salt thereof; glutathione (GSH), preferably reduced L-glutathione; (L-GSH).

In a particular embodiment, the composition comprises Flavine adenine dinucleotide (FAD) or a salt thereof, reduced glutathione (L-GSH), adenosine triphosphate (ATP) or a salt thereof, and may comprise optionally myristic acid, or a salt thereof.

According to a preferred embodiment, the composition of the invention comprises Flavin adenine dinucleotide disodium salt hydrate, reduced L-Glutathione (L-GSH), Adenosine Triphosphate disodium salt hydrate (ATP), and myristic acid or a salt thereof.

In particular embodiments, the vector may be chosen from a metal salt, a polymer, a liposome, a lipid, an excipient, a combination thereof.

In the composition of the invention, the vector may be a polymer selected from any one of poly ethylene glycol (PEG), chitosan, collagen, starch, alginate, hyaluronic acid, a combination thereof, preferably PEG, hyaluronic acid or chitosan, more preferably PEG-600; even more preferably PEG-600-diacid.

The composition of the invention can comprise a metal salt, preferably a gold salt, more preferably HAuCl₄*6H₂0 under a nanoparticule form.

The composition of the invention is provided in a form suitable for intravenous administration, oral administration, sublingual administration, intrarectal administration, intratumoral administration, intraperitoneal administration, intraocular administration, intramucosal administration, intra-vesical, or intra-urethral administration.

In the composition of the invention the dose of FAD can range from 0.01mg to 80 g, the dose of GSH is ranging from 0.1 to 70 g, the dose of ATP is ranging from 0.1mg to 80 g and the dose of myristic acid is ranging from 0.1mg to 10 g. The dose can be given once or several times a day.
The composition of the invention is provided in a therapeutically effective amount and combined to a pharmaceutically acceptable carrier.

The composition of the invention is also provided as a nutraceutical composition in an effective amount and combined to a nutraceutical acceptable carrier. In particular embodiments, the composition is provided to be administered following a posology for reaching a desired effect according to the patient or individual in need thereof, in particular in a patent suffering a side effect due to a medicament or to attenuate a symptom due to a disease.

The composition of the invention is particularly useful to palliate a side effect of a medicament.

Another object of the present invention is a composition of the invention according to any one of the embodiments described here and a drug or a medicament.

Because the therapeutical efficiency of the composition of the present invention alone is demonstrated the composition alone is provided for use in the prevention and/or the treatment of a disease.

The composition of the invention is also provided for use in the prevention and/or the treatment of a disease when combined with another medicament.
In a particular embodiment, the composition of the invention, combined to another medicament is provided for the prevention and/or treatment of a side effect of the medicament.

In a particular embodiment, the composition of the invention, combined to another medicament is provided for the prevention and/or treatment of a side effect of the medicament, and the side effect of the medicament is weight loss.
In particular, the composition of the invention alone or combined to a drug is provided for use in the prevention and/or the treatment of a disease, the disease may be cancer.

The composition of the invention alone or combined to a drug is provided for use as an adjuvant (complement) or neoadjuvant (debulking) to cancer treatment.
In other embodiments, the composition of the invention alone or combined to a drug is provided for its use as an adjuvant or neoadjuvant to cancer treatment.

In particular embodiments, the composition of the invention alone or combined to a drug is provided for use in the prevention and/or the treatment of a cancer wherein the cancer is selected from the group consisting of brain cancer, breast cancer, prostate cancer, lung cancer, airway cancer, upper and/or lower digestive tract cancer, stomach cancer, kidney cancer, pancreas cancer, liver cancer, urinary tract cancer, genital organs cancer, skin cancer, Ear, Nose and Throat (ENT) sphere cancer, and lymphatic organs cancer, preferably pancreatic cancer or liver cancer, more preferably metastatic pancreatic cancer or metastatic liver cancer.
In particular embodiments, the composition of the invention alone or combined to a drug is provided for use in the prevention and/or the treatment of a pancreatic cancer or a liver cancer, especially for use in the prevention and/or the treatment of aggressive forms of pancreatic cancer or of liver cancer.

Under some aspects, the invention is directed to a nutraceutical composition or a pharmaceutical composition, a nutraceutical kit or a pharmaceutical kit.

The composition according to any one of the compositions described here is provided for use in the prevention and/or the treatment of a side effect induced by a drug, preferably a side effect selected from the list consisting of weight loss, nausea, vomiting, headaches, bleeding, hair loss, skin rash, ocular irritation, neutropenia, pain, blood clots, lymphedema, a combination of said side effects, advantageously weight loss.

### BRIEF DESCRIPTION OF THE FIGURES

FIG. 1 represents the effect (means + SEM) of composition A (left panel), or of composition B (right panel), alone or combined to 5-fluorouracil (5-FU) on tumor volume at day 30 (upper panel) and at day 82 (lower panel) after grafting cancer cells, as compared to control group (MODEL), or to 5-FU alone, in a model of metastatic cancer of the pancreas. * P<0.05, ** P<0.01Tukey's test.
FIG. 2 represents the effect of composition A (left panel), or of composition B (right panel), alone or combined to 5-FU on mice survival, as compared to control group (MODEL), or to 5-FU alone, in a model of metastatic cancer of the pancreas.
FIG. 3 represents the Effect of Composition A (high panel), or composition B (low panel), on 5-FU- induced side effect (weight loss), as compared to control group (MODEL), or to 5-FU alone, in a model of metastatic cancer of the pancreas.
FIG. 4 represents the effect of Composition A (right panel), or of composition B (left panel), alone or combined to 5-FU on tumor volume at different times after injection of cancer cells, and at day 19 (lower panels) as compared to control group (MODEL), or to 5-FU alone, in a model of metastatic cancer of the liver.
FIG. 5 represents the effect of Composition A (left panel), or of composition B (right panel), alone or combined to 5-FU on mice survival, as compared to control group (MODEL), or to 5-FU alone, in a model of metastatic cancer of the liver.
FIG. 6 represents the effect of Composition A (high panel), or composition B (low panel), on 5-FU- induced side effect (weight loss), as compared to control group (MODEL), or to 5-FU alone, in a model of metastatic cancer of the liver.

### DETAILED DESCRIPTION OF THE INVENTION

Unless defined otherwise, all technical and scientific terms used herein have the same meaning as commonly understood by a person of ordinary skill in the art.

All amounts in compositions are given as weight-percentages of said substance, unless otherwise stated.

The expression "effective amount", when used to describe therapy to an individual suffering from a disease, refers to the amount of a drug, pharmaceutical agent or active principle of the invention that will elicit the biological or medical response of a cell, tissue, system, animal or human that is being sought, for instance, by a researcher or clinician. Such responses include but are not limited to amelioration, inhibition or other action on a disorder, malcondition, disease, infection or other issue with or in the individual's tissues wherein the disorder, malcondition, disease and the like is active, wherein such inhibition or other action occurs to an extent sufficient to produce a beneficial therapeutic effect. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

"Treating" or "treatment" within the meaning herein refers to an alleviation of symptoms associated with a disorder or disease, or inhibition of further progression or worsening of those symptoms, or prevention or prophylaxis of the disease or disorder, or curing the disease or disorder.

Similarly, as used herein, an "effective amount" or a "therapeutically effective amount" of a compound of the invention refers to an amount of the compound that alleviates, in whole or in part, symptoms associated with the disorder or condition; or halts or slows further progression or worsening of those symptoms or prevents or provides prophylaxis for the disorder or condition. In particular, a "therapeutically effective amount" refers to an amount effective, at dosages and for periods of time necessary, to achieve the desired therapeutic result. A therapeutically effective amount is also one in which any toxic or detrimental effects of compounds of the invention are outweighed by the therapeutically beneficial effects.

### EMBODIMENTS

Before embarking on a detailed review of the products and methods of making the invention, additional features are set out below which may be used in a composition or kit according to the present invention or in a composition or kit according to the present invention in combination with another drug. The various embodiments are as follow:
A composition, hereafter «the composition of the invention» can comprise:
FAD, ATP, and GSH ;
FAD, ATP, GSH, and myristic acid,
FAD, ATP, GSH, and a vector;
FAD, ATP, GSH, myristic acid, and a vector;
(FAD, a vector), ATP;
(FAD, a vector), GSH;
(FAD, a vector), myristic acid;
(FAD, a vector), ATP; GSH;
(FAD, a vector), ATP; myristic acid;
(FAD, a vector), ATP; GSH, myristic acid;

"A vector" or a carrier means a substance, a vehicle, or an excipient; a vector may be a part of the galenic form of an active principle (AP).

AP means a substance on which certain of the properties of a drug depend.

In particular embodiment, a vector comprises a metal salt allowing the composition once into active tumoral cells to be subsequently irradiated in tumors, participating to the destruction of the tumor. An active principle according to the present invention includes but is not limited to: FAD, ATP, GSH and myristic acid, a combination thereof.

Active principles are combined in a composition or are a part of a kit of parts. The dose of each AP is chosen so that they are active alone, and / or in combination with another or all active principle.

In another embodiment, active principles are combined with a medicament in a composition or are a part of a kit of parts. The dose of AP is chosen so that they are active with or on a side effect of a medicament.

The vector according to a particular embodiment can be a polymer, including for example a biopolymer, a metal salt such as a gold salt nanoparticle (eg HAuCl4*6H20), a lipid, a liposome, a combination of vectors or carriers.

In particular embodiments, a polymer can be chitosan, starch, Polyethylene glycol (PEG), hyaluronic acid, a combination of polymers.
The following compositions were found to be active for.
FAD, chitosan, ATP;
FAD, chitosan, GSH;
FAD, chitosan, ATP, GSH;
FAD, chitosan, myristic acid;
FAD, chitosan, GSH, myristic acid;
FAD, chitosan, ATP, myristic acid;
FAD, chitosan, ATP, GSH, myristic acid;
FAD, PEG600, ATP;
FAD, PEG600, GSH;
FAD, PEG600, myristic acid;
FAD, PEG600, GSH, myristic acid;
FAD, PEG600, ATP, myristic acid;
FAD, PEG600, ATP, GSH;
FAD, PEG600, ATP, GSH, myristic acid;
FAD, hyaluronic acid, ATP;
FAD, hyaluronic acid, GSH;
FAD, hyaluronic acid, myristic acid;
FAD, hyaluronic acid, GSH, myristic acid;
FAD, hyaluronic acid, ATP, myristic acid;
FAD, hyaluronic acid, ATP, GSH;
FAD, hyaluronic acid, ATP, GSH, myristic acid;
FAD, gold nanoparticle, ATP;
FAD, gold nanoparticle, GSH;
FAD, gold nanoparticle, ATP ; myristic acid;
FAD, gold nanoparticle, GSH; myristic acid;
FAD, gold nanoparticle, ATP, GSH;
FAD, gold nanoparticle, ATP, GSH, myristic acid;
FAD, gold nanoparticle, PEG600;
FAD, gold nanoparticle, PEG600, myristic acid;
FAD, gold nanoparticle, PEG600, ATP;
FAD, gold nanoparticle, PEG600, GSH;
FAD, gold nanoparticle, PEG600, ATP, GSH;
FAD, gold nanoparticle, PEG600, ATP, GSH, myristic acid;
FAD, HAuCl₄*6H₂0,PEG600;
FAD, HAuCl₄*6H₂0,PEG600, myristic acid;
FAD, HAuCl₄*6H₂0, PEG600, ATP;
FAD, HAuCl₄*6H₂0, PEG600, GSH;
FAD, HAuCl₄*6H₂0, PEG600, ATP, GSH);
FAD, HAuCl₄*6H₂0, PEG600, ATP, GSH, myristic acid;
FAD, HAuCl₄*6H₂0, hyaluronic acid;
FAD, HAuCl₄*6H₂0, hyaluronic acid, myristic acid;
FAD, HAuCl₄*6H₂0, hyaluronic acid, ATP;
FAD, HAuCl₄*6H₂0, hyaluronic acid, GSH;
FAD, HAuCl₄*6H₂0, hyaluronic acid, ATP, GSH;
FAD, HAuCl₄*6H₂0, hyaluronic acid, ATP, GSH, myristic acid.

In any particular embodiments of the above or below embodiments, FAD may be a Flavin adenine dinucleotide disodium salt hydrate, the polymer can be PEG600-diacid or hyaluronic acid; the salt metal, can be a gold nanoparticle, in particular a gold nanoparticle of formula: HAuCl₄*6H₂0.

According to another aspect, the invention concerns a composition, in particular a pharmaceutical composition or a nutraceutical composition.

According to another aspect, the invention concerns a kit (kit of parts).

In another embodiment, the invention concerns a composition, in particular a pharmaceutical composition or a nutraceutical composition for its use in the prevention and/or treatment of a disease.

In another embodiment, the invention concerns a composition, in particular a pharmaceutical composition or a nutraceutical composition and a medicament for its use in the prevention and/or treatment of a disease.

In another embodiment, the invention concerns a composition, in particular a pharmaceutical composition or a nutraceutical composition for its use in the prevention and/or treatment of a side effect of a medicament.

In another embodiment, the invention concerns a composition, in particular a pharmaceutical composition or a nutraceutical composition and a medicament for its use in the prevention and/or treatment of a side effect of the medicament.

A drug may be a medicine or other substance which has a physiological effect when ingested or otherwise introduced into the body:
A Medication means a medicine or a chemical compound used to treat or cure illness.

In a preferred embodiment the disease is a cancer.

The composition of the present invention can comprise a vector that has a protective role for the FAD limiting its degradation by enzymes.

The galenic form makes it possible to improve the absorption and distribution of the combination comprising FAD, GSH, ATP and myristic acid, in the body while limiting its degradation, particularly by blood hydrolases.

According to another aspect, the present invention concerns a composition, advantageously pharmaceutical, in an amount therapeutically effective on cancer comprising FAD in the presence of ATP and GSH, more particularly consisting of an amount of FAD therapeutically effective on cancer in the presence of ATP and GSH and myristic acid.

According to another aspect, the present invention concerns a composition according to the invention, wherein FAD is associated with a biopolymer and/or a metal salt, under the form of a particle, advantageously a nanoparticle and an excipient.

According to another aspect, the present invention concerns a nutraceutical composition, comprising an amount of FAD therapeutically effective on cancer in the presence of ATP and GSH, more particularly consisting of an amount of FAD therapeutically effective on cancer in the presence of ATP and GSH and myristic acid.

According to another aspect, the present invention concerns a nutraceutical composition, comprising an amount of FAD therapeutically effective on cancer in the presence of ATP and GSH, more particularly consisting of an amount of FAD therapeutically effective on cancer in the presence of ATP and GSH and myristic acid, wherein FAD is associated with a biopolymer and/or a metal salt, in the form of a particle advantageously a nanoparticle and an excipient.
In one aspect of the invention, the composition of the invention is provided alone or combined to a medicament.

A medicament combined with the composition or kit of the invention may be any known medicament or drug, in particular drugs that can, as a side effect, affect food intake, appetence, cause nausea, vomiting, headaches, bleeding, hair loss, skin rash, ocular irritation, neutropenia, pain, blood clots, lymphedema, a combination of said side effects.

Depending on the disease to be treated and/or on the side effect of the medicament associated to the composition of the invention to be attenuated, an adequate galenic form of the composition or kit of the invention is provided for a specific route of administration.

The composition can be administered by oral route, intravenous route, intramuscular route, intraperitoneal route, sublingual route, intra ocular route, intranasal route, intra-urethral route, intrarectal route, intertumoral route.

In particular embodiments, each active principle of the composition or the kit according to the invention is provided under a galenic form for a specific route of administration, that may be the same or different from the route of administration of other active principles.
Flavine adenine dinucleotide (FAD)

Flavin adenine dinucleotide (FAD) is a redox-active coenzyme involved with several enzymatic reactions in metabolism.

FAD can exist in four redox states, encompassed here, which are the flavin-N(5)-oxide, quinone, semiquinone, and hydroquinone. FAD is converted between these states by accepting or donating electrons. FAD, in its fully oxidized form, or quinone form, accepts two electrons and two protons to become FADH2 (hydroquinone form). The semiquinone (FADH.) can be formed by either reduction of FAD or oxidation of FADH2 by accepting or donating one electron and one proton, respectively.

In the composition according to the present invention an active principle comprises FAD, ATP, GSH and in particular embodiments myristic acid.

The composition or the kit according to the invention comprises FAD. FAD can exist under the form of a salt in the composition or kit according to the present invention.FAD according to the invention means for example FAD, or a salt of FAD, hydrate or solvate obtained by any one of the methods described in this description or in WO2020089310A1 incorporated by reference here in its entirety. In the composition of the present invention FAD may be naturally protected or protected by combination with a polymer. In other embodiments FAD can be a FAD combined to an enzyme which is modified to be more stable as in JP2015084676A or in EP2844747B1 all incorporated in entirety. In a preferred embodiment FAD can be Flavin adenine dinucleotide disodium salt hydrate as disclosed for example in CN103826642B incorporated by reference here in entirety. A salt of flavin adenine dinucleotide is not particularly limited as far as it is a pharmaceutically acceptable salt, or a salt nutraceutically acceptable.

Examples of salts suitable for any one of the component or AP in the composition of the invention (ie FAD, ATP, myristic acid, hyaluronic acid) include salts with an inorganic acid such as hydrochloric acid, hydrobromic acid, hydroiodic acid, nitric acid, sulfuric acid, or phosphoric acid; salts with an organic acid such as acetic acid, fumaric acid, maleic acid, succinic acid, citric acid, tartaric acid, adipic acid, gluconic acid, glucoheptonic acid, glucuronic acid, terephthalic acid, methane sulfonic acid, lactic acid, hippuric acid, 1,2-ethanedisulfonic acid, isethionic acid, lactobionic acid, oleic acid, pamoic acid, Poly galacturonic acid, stearic acid, tannic acid, trifluoromethanesulfonic acid, benzenesulfonic acid, p-toluene sulfonic acid, lauryl sulfate ester, methyl sulfate, naphthalene sulfonic acid, or sulfosalicylic acid; quaternary ammonium salts with methyl bromide, methyl iodide or the like; salts with a halogen ion such as a bromine ion, a chlorine ion or an iodine ion; salts with an alkali metal such as lithium, sodium or potassium; salts with an alkaline earth metal such as calcium or magnesium; metal salts with iron, zinc or the like; salts with ammonia; and salts with an organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, or N,N-bis(phenylmethyl)-1,2-ethanediamine.

Preferably the salt of FAD is a salt with an alkali metal more preferably the alkali metal is sodium.

As the "salt of FAD" or "salt of ATP", in the present invention, a sodium salt is preferred, and a Flavin adenine dinucleotide disodium salt hydrate is particularly preferred. An adenosine triphosphate disodium salt hydrate is particularly preferred for a salt of ATP.

Thus, Flavin adenine dinucleotide disodium salt hydrate, may be combined to reduced L-Glutathione (L-GSH), and a salt of ATP, such as Adenosine Triphosphate disodium salt hydrate.
In the composition of the invention, FAD or Flavin adenine dinucleotide disodium salt hydrate may be under free form and/or associated with a vector, preferably FAD or Flavin adenine dinucleotide disodium salt hydrate can be associated with at least one vector, more preferably FAD can be associated with at least one vector selected from a metal salt, a polymer, a fatty acid, a combination of vectors.

In the composition of the invention, FAD or Flavin adenine dinucleotide disodium salt hydrate associated with a vector, preferably FAD or Flavin adenine dinucleotide disodium salt hydrate can be associated with hyaluronic acid as in EP2762144 or a polyethylene glycol.

Various metal salts are also suitable for a combination with FAD or Flavin adenine dinucleotide disodium salt hydrate, alone or combined with a polymer, in the composition of the invention. In a preferred embodiment, a combination of FAD-HAuCl4*6H20 or even more preferably of Flavin adenine dinucleotide disodium salt hydrate is provided in the composition of the invention.

The composition or the kit according to the invention comprises Adenosine triphosphate (ATP).

Adenosine triphosphate (ATP) or adenosine-5'-(triphosphate tetra hydrogen), is a well-known compound used in different treatments and as a nutraceutical compound. Various forms and salts of ATP such as those described in US7585850B2, incorporated by reference here in entirety, can be used in combination with FAD and L- GSH, optionally myristic acid, according to the present invention to prepare the composition or kit of the present invention.

The composition or the kit according to the invention comprises Glutathione (GSH).
Reduced Glutathione (GSH) or L- GSH is a tripeptide (glutamic acid-cysteine-glycine) with the formula 2-amino-5 acid - {[2 - [(carboxymethyl) amino] - 1- (mercaptomethyl) -2-oxoethyl] amino} -5-oxopentanoic acid. In its reduced form, GSH represents a strong reducing power. GSH may be produced by any known method or for example as disclosed in EP1391517B1, or in EP2501823, all incorporated herein by reference.

The composition or the kit according to the invention can comprise myristic acid. Myristic acid can be synthesized or extracted by any method or for example as described in EP3146059 or in EP2689020 or in Organic syntheses Coll. Vol. 1 p. 379 (1941) Vol. 6, p.66 (1926).

### CARRIER or VECTOR

In the composition of the present invention FAD combined to a carrier. In particular embodiments the carrier may be a pharmaceutically acceptable carrier or a nutraceutically acceptable carrier. The vector associated with the FAD in the composition of the invention may comprise at least one polymer, preferably at least one biopolymer and more particularly, at least one biopolymer selected from polyethylene glycol (PEG), PEG-diacid, PEG-600, PEG600-diacid; alginic acid or alginate; Poly-Lactide; Bis-Phosphonate; gelatin; maltodextrin; poly amino acids (Poly-L-lysine, Poly-L-ornithine, Poly-L-arginine); lauryl-polyglucose; chitosan; elastin; hyaluronic acid; cellulose; a glucose polymer; and a combination of biopolymers; preferably PEG600, more preferably PEG600-diacid.

In particular embodiments, the vector associated with the FAD comprises alginic acid, collagen, chitosan, a glucose polymer. In particular embodiments, PEG600 or chitosan is associated with FAD in the composition of the invention.

In particular embodiments, the vector associated with the FAD comprises hyaluronic acid in the composition or kit of the invention. A Hyaluronic acid suitable for a nutraceutical or a pharmaceutical composition may be produced by any known method and for example as described in EP3294355 or in WO1992008799A1 incorporated by reference here in.

In an advantageous embodiment of the composition of the invention, FAD is combined to PEG and myristic acid; and ATP, GSH to hyaluronic acid.

In an advantageous embodiment of the composition of the invention, FAD is combined to hyaluronic acid and ATP, GSH to myristic acid.

In particular embodiments, polymer or a biopolymer such as starch, hyaluronic acid, glycogen, sucrose, lactose, a combination of these polymers may be combined to any one of the PA of the composition or kit according to the present invention.

According to the invention, FAD can therefore be associated or combined with any of the glucose polymers selected from starch, glycogen, sucrose, lactose, a combination of several of these glucose polymers in the composition of the invention.

Among the possible collagens associated with FAD in the composition of the invention, collagen I or IV is preferred, even more, preferred human or rabbit collagen I or IV, even more, preferred human collagen I or IV, and even more preferred human collagen I.

In a preferred embodiment a vector comprises PEG-600-diacid.

In another preferred embodiment a vector comprises hyaluronic acid.

### METAL SALT

In particular embodiments, the present invention relates to a composition comprising one among FAD-HAuCl₄*6H₂0, FAD-PEG600, FAD-HAuCl4*6H20-PEG600, FAD-chitosan, FAD-HAuCl4*6H20-chitosan, FAD-human collagen I, FAD-HAuCl4*6H20-human collagen I, FAD-alginate, and FAD-HAuCl₄*6H₂0-alginate, FAD-hyaluronic acid, and FAD-HAuCl₄*6H₂0-hyaluronic acid in combination with ATP, GSH and myristic acid.

FAD-HAuCl₄*6H₂0, FAD-PEG600, FAD-HAuCl₄*6H₂0-PEG600, FAD-chitosan, FAD-HAuCl₄*6H₂0-chitosan are preferred examples of FAD in combination with ATP, GSH and myristic acid according to the invention. More preferred examples of combination in the composition according to the invention are FAD-PEG600, FAD-HAuCl₄*6H₂0-PEG600 or FAD-hyaluronic acid, FAD-HAuCl₄*6H₂0-hyaluronic acid.

In particular embodiments, FAD is protected from degradation using a polymer preferably a biopolymer.

Accordingly, the pharmaceutical or nutraceutical composition according to the invention comprises a FAD-vector compound may be present with ATP, GSH and optionally myristic acid, preferably FAD-vector selected from FAD-polymer, FAD-fatty acid, FAD-biopolymer, FAD-metal salt, and a combination of these compounds FAD-polymer-metal salt, FAD-fatty acid-metal salt, FAD-biopolymer-metal salt.

in other embodiments, FAD is protected from degradation as in EP1034791 incorporated here in its entirety.

The present invention relates in one aspect to a composition comprising a protected FAD-metal salt combination in combination with ATP, GSH and myristic acid in a composition according to the invention and a device providing radiation against cancer.

One object of the present invention may be a FAD-gold salt (FAD-AU salt) or FAD-gold particle combination in combination with ATP, GSH and myristic acid in a composition according to the invention and a device providing infrared, preferably providing near IR.

The composition according to the invention and an infrared (IR) producing device, a device preferably producing near IR is another object of the invention.

Infrared is an electromagnetic radiation; the infrared range is divided into near-infrared (0.5 µm to 5 µm, preferably 0.7 µm < λ < 3 µm), middle infrared (3µ< A < 25 µm m) and far infrared (beyond 25 µm).A near-infrared (0.5 µm to 5 µm) - producing device with any one of the compositions comprising HAuCl4*6H20 described here is an object of the present invention.Solutions comprising FAD, ATP, L-GSH and myristic acid, in free base form, or as pharmacologically acceptable salts may be prepared in water suitably mixed with a surfactant, such as a hydroxypropyl cellulose. Such dispersions according to the present invention may also be prepared in glycerol, liquid polyethylene glycols, and mixtures thereof, and oils. Under normal conditions of storage and use, these preparations contain a preservative to prevent the growth of micro-organisms. The combination of the present invention may be formulated in composition in neutral form or salt form.

"Pharmaceutically" or "pharmaceutically acceptable" refers to molecular entities and compositions which do not cause an adverse reaction, allergic or otherwise, when administered to a mammal, in particular a human being, as appropriate.

Pharmaceutically acceptable "excipient" or carrier means any compound which facilitates the shaping of the pharmaceutical composition and does not alter the nature of the biological activity of the active ingredient(s).

A pharmaceutically acceptable excipient may be a solvent, plasticizer, lubricant, dispersion medium, absorption retarding agent, flow agent, etc. Preferably, the composition further comprises a plasticizer, lubricant, and/or flow agent, pharmaceutically acceptable excipients and optionally sustained-release matrices, such as biodegradable polymers, to form therapeutic compositions.

Pharmaceutically acceptable carrier or excipient refers to a filler, diluent, encapsulating material or solid, semi-solid or liquid formulation auxiliary of any type.

Pharmaceutically acceptable salts include acid addition salts (formed with the free amino groups of the protein) and formed with inorganic acids such as, for example, hydrochloric or phosphoric acids, or organic acids such as acetic, oxalic, tartaric, mandelic acids, etc.

The salts formed with free carboxyl groups can also be derived from inorganic bases such for example, sodium, potassium, ammonium, calcium, or ferric hydroxides, and organic bases such as isopropylamine, trimethylamine, histidine, procaine, and similar.

In the pharmaceutical compositions of the present invention for oral, sublingual, subcutaneous, intramuscular, intravenous, transdermal, local or rectal administration, intratumoral administration, the active principles, alone or in association with another active principle (another drug) potentially exhibiting a side effect, may be administered in a unitary form (or as a kit), mixed with conventional pharmaceutical carriers, or excipients to animals and human beings.

The amount of pharmaceutical excipients in the final products ranges from 1-30%. Appropriate unit dosage forms include oral forms such as tablets, capsules, powders, granules and suspensions or oral solutions, sublingual and oral administration forms, aerosols, implants, subcutaneous, transdermal, topical, intraperitoneal, intramuscular, intravenous, transdermal. intrathecal and intranasal administration forms and rectal, intraurethral administration forms.

The pharmaceutical compositions according to the invention are pharmaceutically acceptable for a formulation capable of being administered orally, sublingually, parenterally, intratumorally, intraperitoneally, subcutaneously, intramuscularly, intravenously, per os (it, ip, sc, im, iv, po), or intraurethral, or intra vesicularly.

These may be, in particular, in sterile, isotonic saline solutions (monosodium or disodium phosphate, sodium, potassium, calcium or magnesium chloride or similar, or mixtures of such salts), or dry compositions, in particular lyophilized, which, depending on the case, of sterilized water or physiological saline, allow the constitution of injectable solutions.

The manufacturing support for meta salt particles containing the composition of the invention can also be a solvent or dispersion medium containing, for example, water, ethanol, polyol (e.g. glycerol, propylene glycol, and liquid polyethylene glycol, etc.), their suitable mixtures, and vegetable oils. Appropriate fluidity can be maintained, for example, by using a coating, such as a lecithin, by maintaining the required particle size in the case of dispersion, and by using a surfactant.

Preventing the action of micro-organisms can be achieved by various antibacterial and antifungal agents, which are well known to the trade. In most cases, it will be preferable to include isotonic agents, for example, sugars or sodium chloride.

Prolonged absorption of injectable compositions can be achieved by the use in the compositions of absorption retarding agents, e.g. aluminum monostearate and gelatine.

Sterile injectable solutions are prepared by incorporating the active principles of the composition or kit of the invention in the required quantity in the appropriate solvent with several of the other ingredients listed herein, as required, followed by sterilization by filtration.

Dispersions according to the invention are prepared by incorporating the sterilized agents of the present invention in a sterile vehicle which contains the basic dispersion medium and the other required ingredients from among those listed above. In the case of sterile powders for the preparation, of sterile injectable solutions, typical preparation processes are vacuum drying and freeze-drying techniques which result in a powder of the combination of FAD, GSH, ATP and myristic acid (or each of these FAD, GSH, ATP and myristic acid individually) and the combination of the present invention plus any additional desired ingredients from a sterile-filtered solution thereof.

Pharmaceutical forms suitable for injectable use include sterile aqueous solutions or dispersions and formulations may comprise, coconut oil, sesame oil, peanut oil, advantageously coconut oil or an aqueous solution of propylene glycol, and sterile powders for the extemporaneous preparation of sterile injectable solutions or dispersions. In any case, the form must be sterile and fluid as long as syringe facilities are available. It must be stable under manufacturing and storage conditions and must be preserved from the contaminating action of micro-organisms, such as bacteria and fungi.

### NUTRACEUTICAL COMPOSITION

The invention relates under another main aspect to a nutraceutical composition of the invention comprising FAD, GSH, and ATP, optionally myristic acid, in an effective amount and a nutraceutical excipient.

Another aspect of the invention relates to a drug, in particular a drug used at a dose inducing a side effect and the nutraceutical composition of the invention.

Nutraceutical excipients are inactive ingredients used during the production of nutraceuticals. Some of these ingredients include film coating, thickeners, preservatives, lubricants, binders, and colors, among others.

The composition of nutraceutical excipients in the final products ranges from 0.1-30%. Unless otherwise specified, all percentages and amounts expressed herein and elsewhere in the specification should be understood to refer to percentages by weight.

As used herein, "an effective amount", and like terms refer to that amount of a compound, material or composition as described herein that may be effective to achieve a particular result. Such results may include, but are not limited to, the reduction of inflammation, reduction of tumor growth, increase of survival, reduction of side effects of a medicament. Such effective activity may be achieved, for example, by administration of compositions of the present invention to an individual. An effective amount may be based on several factors, including weight and frequency of treatment or consumption of the compositions of the present invention, e.g., once, twice, or three times daily, and other drugs given to the individual.

### DOSES

Whether in a pharmaceutical or a nutraceutical composition or kit according to the present invention, a dose of AP (FAD, GSH ATP, myristic acid) may be as follow.

### Doses of FAD

The amount of FAD in the composition or the kit of the present invention may be comprised between 0.1mg and 50 g, in particular from 1 mg to 500 mg, the composition may be taken once, twice or three times a day. In a particularly preferred embodiment the dose of FAD is 25 mg/kg. In a particularly preferred embodiment the dose of FAD is 25 mg/kg every day or every two days.ln a particularly preferred embodiment the dose of FAD is given daily or every two days for at least a month or at least 2 months, or at least 3 months or at least 6 months. Doses of ATPThe quantity of ATP in the composition or the kit of the present invention according to the invention may be expressed in mM and the dose administered may be between 0.1mM and 10 mM, or between 0.5 mM to 1 mM.

The quantity of ATP in the composition or the kit of the present invention according to the invention may also be expressed in g, mg or µg and the dose administered by weight.

An effective amount of ATP may also be provided at a dosage level of 100mg/dose.
ATP may be used at a dose of 100 mg, for a local concentration of approximately 1 to 10 mmol / L or higher.

Advantageously, the dose of ATP according to the invention can vary from 1 mg to 1000 mg/kg, preferably from 10 to 100 mg/kg, for example 400 mg/kg or 100 mg/kg.

As an example, the sublingual form of ATP under the name of ATEPADENE may be used in an individual with an average weight of 60 to 80 kg. ATEPADENE at a dose of 30 mg/capsule is an interesting source of ATP for its use according to the invention, or else ATP Max(R) 400 mg or PEAK ATP(TM).

An effective amount of ATP is provided at a dosage level ranging from 0.01 mg/kg to about 1 g/kg (of body weight) per day, especially about 0.1 mg/kg to 100 mg/kg of body weight per day; preferably 100 mg/kg 3x/d or 100mg/kg 5x/d; 250 mg/kg 2x/d or 250 mg/kg 3x/d. Under the active sublingual form, ATP may be taken at a dose of 3 times 30mg.

In a particularly preferred embodiment, the dose of ATP is 25 mg/kg.

In a particularly preferred embodiment the dose of ATP is given daily or every two days for at least a month or at least 2 months, or at least 3 months or at least 6 months.

### Doses of GSH

The dose of GSH in the composition or the kit of the present invention is ranging from 0.01 mg to 5 g, preferably a dose in an amount of 100 mg; or 500mg can be used in the composition of the kit of the present invention. GSH may be taken one to 3 times a day.

Advantageously, the GSH in the composition according to the invention can be administered at a dose ranging from 0.01 mg to 10 g, from 0.1 mg to 1 g, from 1 mg to 1 g, from 10 mg to 900 mg, from 100 mg to 300 mg, at a dose of 100 mg/dose or mg/kg or 200 mg/dose or per mg/kg.

The dose of reduced Glutathione administered per dose in the composition according to the invention, for example by the sublingual route, is 100 mg, 200 mg or 300 mg or 40 mg (Sublinthion).

GSH may be present in quantities ranging from 0.01 mg to 10 g, from 0.01 mg to 1 g, from 0.1 mg to 1 g, 1 mg to 10 mg, from 40 mg to 900 mg, from 100 mg to 300 mg, 100mg or 200mg.

In a particularly preferred embodiment, the dose of L- GSH is 25 mg/kg.

In a particularly preferred embodiment, the dose of GSH is given daily or every two days for at least a month or at least 2 months, or at least 3 months or at least 6 months.

### Dose of Myristic acid.

In general, a suitable dose of myristic acid may be in the range of from about 0.5 to about 100 mg/kg, e.g., from about 1 to about 75 mg/kg of body weight per day, or 1.5 to about 50 mg per kilogram body weight of the recipient per day, or about 2 to about 30 mg/kg/day, or about 2.5 to about 15 mg/kg/day. Myristic acid can be conveniently administered in unit dosage form; for example, containing 5 to 1000 mg, conveniently 10 to 750 mg, most conveniently, 50 to 500 mg of active ingredient per unit dosage form.

Myristic acid can be administered to achieve peak plasma concentrations of the active compound of from about 0.5 to about 75 µM, preferably, about 1 to 50 µM most preferably, about 2 to about 30 µM This may be achieved, for example, by the intravenous injection of a 0.05 to 5% solution of the active ingredient, optionally in saline, or orally administered as a bolus containing about 1-100 mg of the active ingredient.

Desirable blood levels may be maintained by continuous infusion to provide about 0.01-5.0 mg/kg/hr. or by intermittent infusions containing about 0.4-15 mg/kg of the active ingredient(s).

In a particularly preferred embodiment, the dose of myristic acid is administered is 10 mg/kg.

In a particularly preferred embodiment, the dose of myristic acid is given daily or every two days for at least a month or at least 2 months, or at least 3 months or at least 6 months.

The invention provides a method for preparing a composition according to the present invention, said method comprising
- combining a polymer, preferably PEG 600 or hyaluronic acid with FAD, optionally with a metal salt,
by adding a polymer, preferably PEG 600 or hyaluronic acid into a FAD solution (5mg/ml), then combining the resulting FAD-polymer (and/or metal salt) with L-GSH, (5mg/ml), ATP (5mg/ml) and optionally myristic acid. (2mg/ml).

The proportion of FAD, ATP, GSH may be 1:1:1 depending on the dosage and form of the molecule used it can be adapted or variate but not less than a proportion 1:50 or 50:1. This is for ensuring a synergistic effect of the molecules on cell proliferation and /or differentiation.

The composition or the kit of the invention is provided to be used alone for preventing and/or treating a disease, or a side effect.

According to the results obtained here, the composition of the invention is also provided for use in the prevention or the treatment of any disease, in particular a disease or a symptom or group of symptoms due to a medication or a drug.

A "disease" finds a possible definition in : a disorder or structure orfunction in a human, animal, especially one that has a known cause and/or a distinctive group of symptoms, signs, or anatomical changes of said .

The composition of the invention is provided for use alone for preventing and/or treating a disease, in particular disease associated with inflammation such as cancer, or neurodegenerative diseases. In preferred embodiments the composition of the invention is provided for use alone for preventing and/or treating a cancer.

The cancer may be any cancer, or metastatic cancer, or cancer with a fast progression (aggressive).

An aggressive cancer may be for example a cancer progressing fast due to overexpression of Aurora kinase A (AURKA) and/or B (AURKB) as compared to healthy cells.

The composition of the invention is provided for preventing and/or treating a cancer as an adjuvant or neoadjuvant to cancer treatment.

Adjuvant chemotherapy refers to chemotherapy that people receive after another cancer treatment, such as surgery. Neoadjuvant chemotherapy is when a person has chemotherapy as their main treatment before other options. Both adjuvant and neoadjuvant therapies may encompass chemotherapy, radiation therapy, hormone therapy, and others. Neoadjuvant therapy occurs prior to someone's primary cancer treatment. Adjuvant therapy occurs after. For example, an individual may have surgery to remove a tumor, followed by adjuvant chemotherapy.

The composition of the invention is provided for preventing and/or treating a cancer as an adjuvant and neoadjuvant to cancer treatment. The composition of the invention is provided for use in combination (during, before and after) cancer treatment whether cancer treatment is used as adjuvant or neoadjuvant.

### KIT

The invention also provides a kit comprising Flavin adenine dinucleotide disodium salt hydrate combined to a polymer, or a FAD more resistant to degradation as disclosed above, Adenosine Triphosphate disodium salt hydrate (ATP), L- GSH as another object of the present invention.

Advantageously the composition or kit can also be brought into the presence of a fatty acid, preferably, a fatty acid selected from myristic acid, palmitic acid, oleic acid, a combination thereof, more preferably myristic acid.

The invention also provides a kit comprising Flavin adenine dinucleotide disodium salt hydrate combined to a polymer, or a FAD more resistant to degradation as disclosed above, Adenosine Triphosphate disodium salt hydrate (ATP), L- GSH and myristic acid as another object of the present invention.

Under one preferred aspect the invention is directed to a composition or a kit of the invention comprising fatty acids with particular fatty acids that can form a liposome or a capsule comprising an encapsulated composition of the invention comprising FAD, ATP, GSH and optionally, myristic acid.

Myristic acid may be obtained from extraction of natural sources or synthesis as in EP2861059.

An aspect of the invention is that a composition according to the invention is formulated in a form suitable for parenteral administration including intravenous, intramuscular, and subcutaneous administration, mucosal administration e.g. oral, intranasal, vaginal, or rectal, is an aspect of the invention.

Other aspects of the invention are a composition according to the invention in a form suitable for intra bladder or intraurethral administration.

According to another aspect, the present invention concerns a pharmaceutical composition or a kit, advantageously pharmaceutical, comprising a salt of FAD, of ATP, L-GSH and optionally myristic acid in a therapeutically effective quantity, and at least one other medication.

According to another aspect, the present invention concerns a nutraceutical composition or a kit, advantageously pharmaceutical, comprising a salt of FAD, of ATP, L-GSH and optionally myristic acid in a therapeutically effective quantity, and at least one other medication.

According to another aspect, the present invention concerns a pharmaceutical composition or a kit comprising a salt of FAD, of ATP, L-GSH and optionally myristic acid according to the invention in a therapeutically effective quantity, for its use in combination with surgery and/or radiotherapy, preferably in combination with exeresis and/or rays, more preferably in combination with exeresis and/or near-infrared rays.

According to another aspect, the present invention concerns a pharmaceutical composition or a kit comprising a salt of FAD, of ATP, L-GSH and optionally myristic acid according to the invention in a therapeutically effective quantity, and at least one other medication for its use in combination with surgery and/or radiotherapy, preferably in combination with exeresis and/or rays, more preferably in combination with exeresis and/or near-infrared rays.

According to another aspect, the present invention relates to a pharmaceutical composition or kit, and at least one drug or medicament used in chemotherapy, preferably in anti-cancer chemotherapy.

The invention relates in one aspect to a combination of the composition of the invention and at least one anti-cancer chemotherapeutic agent.

A drug used in anti-cancer chemotherapy associated with the composition of the invention may be selected from a chemotherapeutic agent, a drug used in hormone therapy, a drug used in immunotherapy, a drug used in targeted immunotherapy.

Among chemotherapeutic agents against cancer that be be used with the composition or kit according to the invention, the drug used in anticancer chemotherapy associated with the composition of the invention may be:
- a microtubule active agent;
- ii. an alkylating agent;
- iii. an anti-metabolite agent;
- iv. an intercalating agent; v. an inhibitor of topoisomerase I or II; such as camptothecin and its derivatives;
- vi. a compound targeting / reducing protein or lipid activity or protein or lipid phosphatase activity; vii. a proteasome inhibitor;
- viii. a protein kinase inhibitor;
- ix. an antibiotic,
- x. an anti-inflammatory agent;
- xi. a monoclonal antibody.

According to another aspect, the present invention concerns a pharmaceutical composition or kit, and at least one drug used in cancer chemotherapy, for use in the treatment of cancer.

According to another aspect, the present invention concerns a pharmaceutical composition or kit, and at least one drug used in cancer chemotherapy, for use in the treatment of side effects due to anti-cancer treatment.

Anti-cancer chemotherapy is a general drug-based treatment aimed at destroying cancer cells or preventing them from multiplying throughout the body. There are many different chemotherapy drugs, often combined to increase the effectiveness of the treatment.

According to one aspect, the composition of the invention can be used:
- in an ancillary situation, once the tumor has been removed, ensure that no cancer cells remain.
- In a neo-adjuvant situation, for reducing the size of the tumor before surgery: makes it possible to limit the potential sequels related to surgery.
- In case of metastatic disease, to treat the whole body.
- To prevent cancer development.

There are examples of chemotherapies, which are used depending on the characteristics of cancer.

According to one aspect, the invention relates to the composition according to the invention, used before, during, and/or after i) exeresis surgery, preferably an tumor excision, ii) radiotherapy, preferably IR, iii) chemotherapy, preferably anti-cancer chemotherapy, preferably classical (anti-cancer) chemotherapy such as chemotherapy using synthetic or plant-derived products, hormone therapy, immunotherapy, targeted immunotherapy.

An exeresis is a surgical operation consisting of removing from the body an element that is harmful or useless to it (organ, tumor, foreign body, etc.).

Immunotherapy aims to help or replace the immune system to recognize and attack pathological cells, particularly cancerous cells; some seek to bypass the mechanisms that prevent Tor B lymphocytes from triggering an immune response, while others seek to stimulate the specific immune response.

Immunotherapy involves treating a patient with an immunity-derived compound, such as an antibody, fusion protein, receptor, preferably an antibody, and more preferably a monoclonal antibody.

Among the chemotherapy drugs or agents that can be associated with the composition according to the invention, a synthetic or plant-derived product, a hormone-based drug, comprising a hormone agonist or antagonist, a drug comprising an immunity-derived protein, such as a therapeutic antibody, or an immune cell selected or modified to act specifically on a cellular or tissue target.

According to another aspect, the present invention relates to a pharmaceutical composition or kit comprising an excipient and a salt of FAD, of ATP, L-GSH, optionally of myristic acid, in a therapeutically effective amount, and at least one therapeutic antibody, preferably a monoclonal antibody.

The term "monoclonal antibody" as used here includes, but is not limited to, bevacizumab, cetuximab, trastuzumab, Ibritumomab tiuxetan, rituximab and tositumomab, and iodine 131.

Bevacizumab may be administered in its commercial form, e.g., AVASTIN; cetuximab as ERBITUX; trastuzumab as HERCEPTIN; rituximab as MABTHERA; ibritumomab tiuxetan as ZEVULIN; and tositumomab and iodine 131 as BEXXAR.

Monoclonal therapeutic antibodies that may be combined with the composition according to the invention include Rituximab, ATC Code L01XC02, Trastuzumab (Herceptin), ATC Code L01XC03, Gemtuzumab ozogamicin, ATC code L01XC05, Alemtuzumab, ATC code L01XC04, Ibritumomab tiuxetan, ATC code V10XX02, Cetuximab, ATC code L01XC06, Bevacizumab, ATC code L01XC07, Nivolumab, Ipilimumab are particularly suitable.

These monoclonal antibodies are used in the treatment of cancer and therefore can be used in combination with the composition according to the invention, at doses lower than those conventionally used.

Hormone therapy is understood to be a therapy based on the administration of a drug, for example, a hormone, steroid antagonist or agonist.

According to a preferred aspect, the present invention relates to a pharmaceutical composition or kit comprising (a) the combination of the invention in a therapeutically effective amount, and (b) at least one drug used in hormone therapy.

The invention relates in one aspect to a combination of the composition of the invention and at least one chemotherapeutic agent selected from anti-metabolite, an antibiotic, an alkylating agent, an intercalating agent (anthracyclines), a microtubule inhibitor, a topoisomerase I or II inhibitor, a spindle poison (taxoid, vinca-alkaloid, an oxazaphosphorin), a proteasome inhibitor, a protein kinase or phosphatase inhibitor.
Examples are provided for i) a Topoisomerase I inhibitor, such as Irinotecan, topotecan ii) a Topoisomerase II inhibitor, such as an anthracycline or an Epidodophyllotoxin (etoposide), iii) a spindle poison such as a Taxane e.g. docetaxel, paclitaxel, a Periwinkle alkaloid such as vincristine, vinblastine, vindesine, vinorelbine, iv) an antimetabolite, such as an antifolate like methotrexate MTX, Pemetrexed; a purine analog-like fludarabine; a pyrimidine analog, like, 5-fluorouracil (5-FU), gemcitabine, cytarabine, v) an alkylating agent selected from nitrogen mustard, nitrosourea, alkylsulphonate, and sulfate (such as methyl triflate and dimethyl sulfate), a platinum salt such as trimethyloxonium tetrafluoroborate, cysplatinum, carboplatinum, oxaliplatinum, an oxazaphosphorine such as cyclophosphamide, ifosfamide, fluorouracil, or 5-fluorouracil (5-FU), may be used in the treatment of cancer with the composition of kit of the invention.

Term "anti-metabolite" or "anti-neoplastic anti-metabolite" includes, but is not limited to, 5-fluorouracil (5-FU); capecitabine; gemcitabine; DNA demethylation agents such as 5-azacytidine and decitabine; methotrexate; edatrexate; and folic acid antagonists such as, but not limited to, pemetrexed. Capecitabine may be administered, for example, in the form in which it is marketed, for example, under the brand name XELODA; and gemcitabine in the form of GEMZAR.

Anti-metabolites interfere with DNA synthesis of constituents; they are structural analogs, on the one hand, of purine and pyrimidine bases (or corresponding nucleosides) and, on the other hand, of folinic coenzymes, as the latter are involved in numerous stages of purine and pyrimidine biosynthesis. Their action is to inhibit cell replication, e.g. by incorporation into nucleic acids, which leads to cell death including DNA breakdown.

Among the antimetabolites that can be associated with the composition of the invention, i) Pyrimidine analogs such as 5-FU, tegafur, capecitabine, azacitidine, gemcitabine ; ii) Analogues of purines such as mercaptopurine, fludarabine, azathioprine, cladribine, pentostatin, cytarabine, nelarabine, clofarabine. iii) Folic acid analogs such as methotrexate, pemetrexed, pralatrexate, ralitrexed, trimetrexate, piritrexine iv) decitabine, sapacitabine are particularly suitable.

An antimetabolite associated with/to the composition or kit according to the invention is selected from 5-FU, Cytarabine, Capecitabine (xeloda), Fluoropyrimidine (Alimta), pemetrexed, Gemcitabine (gemzar), Tomudex (raltitrexed), more preferably 5-FU.

The composition or kit according to the invention can be combined with an antifolate, methotrexate, or pemetrexed and or with a purine analog, fludarabine, or a pyrimidine analog, 5-FU.

5-FU belongs to the class of anti-metabolite drugs, a subclass of pyrimidine analogs.

The invention concerns a composition of the invention and an anti-metabolite drug, preferably an anti-metabolite drug such as 5-fluorouracil (5-FU).

The invention furthermore concerns, according to a preferred method of production, pharmaceutical compositions comprising:
(a) a therapeutically effective amount of the composition according to the invention;
(b) an antimetabolite, preferably a pyrimidine analog and more preferably 5-FU,
(c) a pharmaceutically acceptable carrier.
Furthermore, the present invention concerns, according to a preferred method of realization, a kit or a commercial product comprising:
(a) a pharmaceutical formulation or a neutraceutical formulation of the composition of the invention,
(b) a pharmaceutical formulation of one or more chemotherapeutic agents;
(a) and (b) for simultaneous, concurrent, separate, or sequential use.

In particular embodiments, the posology for combining the composition of present invention to a drug or a medication may be a daily administration or an administration every two day of the composition regardless of the posology of the anti-cancer drug, preferably an administration allowing the level of FAD, ATP, GSH to be constant as compared to a level without administration of the composition of the invention comprising FAD ATP GSH, myristic acid (ie only due to endogenous of FAD ATP GSH).

The length of the administration of the composition is at least the same as that of other treatments and/or longer, to maintain the level of FAD, ATP, GSH constant as compared to a level without administration of the composition of the invention comprising FAD ATP GSH, myristic acid (only due to endogenous of FAD ATP GSH).

Furthermore, the present invention concerns, according to a preferred method of realization, a kit or a commercial product comprising:
(a) a pharmaceutical formulation of FAD-PEG, ATP, GSH and myristic acid,
(b) a pharmaceutical formulation of one or more chemotherapeutic agents comprising an antimetabolite, preferably a pyrimidine analog and more preferably 5-FU,
(a) and (b) for simultaneous, concurrent, separate, or sequential use.

A standard or conventional chemotherapeutic agent combined with the composition according to the invention is an object of the invention.
The term "conventional chemotherapeutic agents" is broad and encompasses many chemotherapeutic agents with different mechanisms of action. The combination of these with the composition or kit of the invention improves the treatment of cancer. As a general rule, chemotherapeutic agents are classified according to their mechanism of action.

The term "microtubule active agent", or "spindle poison" as used here, refers to microtubule stabilizing agents, microtubule stabilizers, and microtubule polymerization inhibitors, including, without limitation, taxanes, e.g. paciltaxel and docetaxel; vinca-alkaloids, e.g. vinblastine, in particular, vinblastine sulfate; vincristine, in particular, vincristine sulphate and vinorelbine; discodermolides; cochicine and epothilones and derivatives thereof, e.g. epothilone B or a derivative thereof. These "microtubule active agent", or "spindle poison" as used here to be combined with the composition or kit of the invention.

Paclitaxel is marketed as TAXOL; docetaxel as TAXOTERE; vinblastine sulfate as VINBLASTIN R.P; and vincristine sulfate as FARMISTIN. Generic forms of paclitaxel are also included, to be used with the composition or kit of the invention as well as various galenic forms of paclitaxel. Generic forms of paclitaxel include but are not limited to, betaxolol hydrochloride. Various dosage forms of paclitaxel include, but are not limited to, paclitaxel, an albumin nanoparticle, marketed as ABRAXANE; ONXOL, CYTOTAX. Discodermolide can be obtained, for example, as described in US Patent No. 5010099.

Besides, the epotholine derivatives which are described in US Patents No. 6.194.181, WO 98/10121, WO 98/25929, WO 98/08849, WO 99/43653, WO 98/22461, and WO 00/31247 are also included; epotholine A and/or B in particular, may be combined with the composition or kit of the invention.

Among the preferred spindle poisons combined with the composition or kit of the invention the following vincristine, vinblastine, vinorelbine, vindesine, vinorelbine, vinflumine; ii) dolastatin, such as romidepsine; Paclitaxel, nab, paclitaxel, docetaxel, carbitaxel; epothilone, Ixabepilone, are particularly suitable.

The most preferred spindle poisons in combination with the composition or kit of the invention are selected from ocetaxel, paclitaxel, vincristine, vinblastine, vindesine, vinorelbine.

A more preferred spindle poison in combination with the composition the invention is selected from Taxol, Docétaxel, Paclitaxel, Vincristine, Vinorelbine, Vindesine, and Vinblastine (Velbe).

The spindle poisons selected from Taxol, Taxotere (docetaxel), Paclitaxel, Vincristine are the most preferred in combination with the composition of the invention.
Eribulin (Halaven) is a microtubule inhibitor. It is a synthetic analog with a simplified structure of halichondrin B, a substance isolated from the marine sponge Haichondria okadai.
Eribulin in combination with the composition or kit of the invention are an object of the present invention.

The term "alkylating agent", as used herein, includes, but is not limited to, i) Nitrogenous mustards (melphalan, chlorambucil, estramutine; ii) Oxazaphosphorines, cyclophosphamide, ifosfamide; iii) Triazenes and hydrazines, procarbazine, dacarbazine, temozolomide; iv) Ethylene imines, aziridines, thiotepa, mitomycin C; v) Nitrosoureas BCNU, CCNU, fotemustin, streptozotocin; vi) Alkyl alkanes sulphonate, busulfan; vii) Organoplatins or "platinum compound".

Any of these alkylating agents or several of them can be combined to the composition or kit of the invention.

The term "platinum compound" as used herein includes, but is not limited to, carboplatin, cisplatin, cisplatin, oxaliplatin, satraplatin, and platinum agents such as ZD0473. Carboplatin may be administered, for example, in the form in which it is marketed, e.g., CARBOPLATE; and oxaliplatin as ELOXATIN.

Trabectedine can be also combined with the composition or kit of the invention.
An intercalant selected from Melphalan, chlorambucil - BCNU - a platinum salt - an aziridine,: mitomycin C can be combined with the composition of the invention.

Within the intercalants, an anthracycline such as Doxorubicin ADRIBLASTINE^{®} Epi or Adriamycin FARMORUBICINE^{®} or Idarubicin ZAVEDOS^{®} is particularly well adapted for use in combination with the composition or kit of the invention.

According to another embodiment, the composition of the invention can be associated and given to a patient with any of the intercalants selected from cyclophosphamide, ifosfamide, Imelphalan, BCNU (or Gliadel), temozolamide (TEMODAR), Cisplatinum, Carboplatinum, free platinum or in salt form, Bendamustine, and Temodal.

Cyclophosphamide can be administered, with the composition of the invention, for example, in the marketed form CYCLOSTIN; and ifosfamide as HOLOXAN.

According to a preferred embodiment, the composition of the invention is combined with Cyclophosphamide (endoxan).

According to a preferred embodiment, the composition or kit of the invention is combined with Cyclophosphamide (endoxan) and another drug.

According to a preferred embodiment, the composition or kit of the invention is combined with Bendamustine.

According to a preferred embodiment, the composition or kit of the invention is combined with Temodal.

Anti-metabolites interfere with DNA synthesis; they are structural analogs, on the one hand, of purine and pyrimidine bases (or corresponding nucleosides) and, on the other hand, of folinic coenzymes, as the latter are involved in numerous stages of purine and pyrimidine biosynthesis. Antimetabolites are very powerful but often associated with strong side effects.

Accordingly, the composition or kit of the invention is particularly suited to be combined to an anti-metabolite.

As a main object, the invention provides any of the compositions or kit of the invention disclosed here for palliating a side effect induced by a drug or a symptom due to a disease, preferably weight loss.

A side effect means a secondary, typically undesirable effect of a drug or medical treatment.

The drug inducing a side effect, or several side effects, can be any drug.

By definition, a drug or a medication has a therapeutic effect in a population of patients and can have one or several side effects.

An appropriate dose of, e.g. 5-FU in human is an appropriate dose decided by a medical doctor, it may be in the range of 100-1500mg per day, e.g. 200-1000mg/day, such as 200, 400, 500, 600, 800, 900, or 1000mg/day, administered in one or two doses per day. 5-FU can be administered to a human being in a dosage range of approximately 50-1000mg / m 2 / day, e.g. 500mg / m 2 / day.

Dosages of 5-FU can be reduced when combined with the composition of the invention. A reduction of 10% to 50% is preferred.

Such doses may be reduced by at least 10% in combination with the composition of the invention.

Such doses may be reduced by at least 0% in combination with the composition or kit of the invention and at least one side effects due to reduced.

Olaparib acts by inhibiting poly (ADP-ribose) polymerases (PARP). The composition of the invention may be combined with Olaparib.

The term "proteasome inhibitor", as used here, includes compounds that target, decrease or inhibit the activity of the proteosome. Compounds that target, reduce, or inhibit proteosome activity include, but are not limited to PS-341; MLN 341, bortezomib, or velcade.

The composition or kit of the invention may be combined with bortezomib.

The term "topoisomerase I inhibitor", as used herein, includes Irinotecan, topotecan, camptothecin, and its active derivatives.

Camptothecin acts by interfering with the unfolding of DNA super wound by the cellular enzyme topoisomerase I, which triggers events leading to apoptosis and programmed death in malignant cells.

The topoisomerase I inhibitor Campto (irinotecan) in combination with the composition according to the invention as a pharmaceutical combination or a kit according to the invention.

The term "topoisomerase II inhibitor" as used here includes, but is not limited to, Anthracyclines (intercalants), doxorubicin, daunorubicin, epirubicin, idarubicin, and nemorubicin; Anthracenediones, anthraquinones, mitoxantrone, and losoxantrone; epidophyllotoxins, etoposide, teniposide; or amsacrine or even bleomycin; Epidodophyllotoxins (etoposide).
Etoposide has been marketed as ETOPOPHOS; teniposide as VM 26-BRISTOL; doxorubicin as ADRIBLASTIN or ADRIAMYCIN; epirubicin as FARMORUBICIN; idarubicin asZAVEDOS; and mitoxantrone as NOVANTRON. The daunorubicin, includes the liposomal formulation, e.g. DAUNOSOME;
Doxorubicin is preferred in combination with the composition of the invention. Doxorubicin includes the liposomal formulation, e.g. CAELYX;

Epirubicin is preferred in combination with the composition of the invention depending on the invention.

Etoposide, marketed as ETOPOPHOS, is a preferred in combination with the composition of the invention.

A topoisomerase II inhibitor selected from Etopophos (etoposide), topotecan, innotecan in combination with the composition according to the invention is a pharmaceutical combination or a kit according to the invention.

A topoisomerase II inhibitor selected from Etopophos (etoposide), topotecan, innotecan in combination with the composition of the invention is a pharmaceutical combination or a kit according to the invention.

Bricatinib is an inhibitor of ALK ("Anaplastic lymphoma kinase") and EGF receptor;
Bricatinib and the composition of the invention or Bricatinib and the composition of the invention, constitute a pharmaceutical combination or a kit, depending on the invention.

Palbociclib (Ibrance) is a molecule that inhibits two cyclin-dependent kinases, CDK4 and CDK6, and are proteins necessary for the cell cycle.

Palbociclib and any of the compositions of the invention described herein constitute a pharmaceutical combination or a kit according to the invention.

Sorafenib (Nexavar) is a tyrosine kinase inhibitor. FAD and Sorafenib or Sorafenib and the composition according to the invention, constitute a pharmaceutical combination or a kit according to the invention.

The preferred classical chemotherapy drugs for combination with anyone of the compositions according to the invention are selected from any of the following classical chemotherapy drugs: Taxol, Taxotere (docetaxel), Paclitaxel, 5-FU, Etopophos (etoposide), Doxorubicin, Vincristine, Capecitabine (xeloda), Vinblastine (Velbe), Cyclophosphamide (endoxan), Alimta (pemetrexed), Cytarabine, Cisplatin, Carboplatin, Free Platinum, Olaparib, Epirubicin, Campto (irinotecan), Gemcitabine (gemzar), Bricatinib, Eribulin (Halaven), Bortezomib (Velcade), Bendamustine, Palbociclib (Ibrance), Temodal, Sorafenib (Nexavar), Tomudex (raltitrexed), Letrozole (anti-aromatase, complementary hormone therapy); a combination of these drugs.
According to a particular mode of realization, the usual doses (prescribed by a physician or medical doctor) of conventional anti-cancer chemotherapy drug can be increased, and/or the side effects caused by conventional anti-cancer chemotherapy drug be greatly reduced when the conventional anti-cancer chemotherapy drug is combined with the composition or kit according to the invention (compared to side effects observed without the composition or kit according to the invention). Accordingly, the length of conventional anti-cancer chemotherapy can be reduced, and results obtained unchanged or improved.

According to a particular mode of realization, the usual doses (prescribed by a physician) of conventional anti-cancer chemotherapy drug can be unchanged, and the side effects greatly reduced when the conventional anti-cancer chemotherapy drug is combined with the composition or kit according to the invention.

According to other particular method of realization, the usual doses (prescribed by a doctor) of conventional cancer chemotherapy drugs can be reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% when the conventional cancer chemotherapy drug is combined with the FAD, ATP, GSH, myristic acid, according to the invention; the anticancer activity of the combination with the composition of the invention is equivalent or superior to that measured with 100% of the active dose of the conventional anti-cancer chemotherapy drug.

The combination of the invention may also be applied in combination with other treatments, for example, surgery, hyperthermia, immunotherapy, and/or radiation therapy.

### DISEASE

The composition or the kit of the invention is provided as a medicament or a dietary complement.

The composition or the kit of the invention for its use according to any of the foregoing methods of therapeutical action or for reducing side effect of a drug, is disclosed.

The composition or the kit of the invention is provided as a medicament for use in the treatment or prevention of a disease.

A disease or condition significantly impacted by the treatment of the present invention may be selected from the group which includes, e.g., inflammation; infection; atherosclerosis; hypertension; cancer; radiation injury; neurological disease; neurodegenerative disease; ischemia/reperfusion injury; aging; wound healing; glutathione deficiency; acquired immunodeficiency syndrome; sickle cell anemia; and diabetes mellitus.

The compositions of the present invention are useful to prevent or treat the following disorders and diseases: memory loss; Parkinson's disease; aging; toxin-induced hepatotoxicity; inflammation; liver cirrhosis; chronic hepatitis; and diabetes due to cirrhosis; indigestion; fatigue; stress; cough; infertility; tissue inflammation; cancer; anxiety disorders; panic attacks; rheumatism; pain; manic depression; alcoholic paranoia; schizophrenia; fever; insomnia; infertility; aging; skin inflammations and disorders; alcoholism; anemia; carbuncles; convalescence; emaciation; HIV; AIDS; immune system problems; lumbago; multiple sclerosis; muscle energy loss; paralysis; swollen glands; ulcers; breathing difficulties; inflammation; psoriasis; cancer (e.g.; prostate cancer, lung cancer and breast cancer); pain; cardiovascular disease (e.g.; arteriosclerosis and atherosclerosis); ischemia/reperfusion injury; anxiety; attention deficit disorder; leprosy; arthritis (e.g., psoriatic arthritis; ankylosing spondylitis; and rheumatoid arthritis); hemorrhoids; tuberculosis; high blood pressure; congestive heart failure; venous insufficiency (pooling of blood in the veins; usually in the legs); sore throat; hepatitis; syphilis; stomach ulcers; epilepsy; diarrhea; asthma; burns; piles; sunburn; wrinkles; headache; insect bites; cuts; ulcers; sores; herpes; jaundice; bursitis; canker sores; sore gums; poison ivy; gastritis; high cholesterol; heart disease; bacterial infection; viral infection; acne; aging; immune disorders; dental caries; periodontitis; halitosis; dandruff; cardiovascular disease (e.g., hypertension; thrombosis; arteriosclerosis); migraine headaches, diabetes; elevated blood glucose; diseases of the alimentary canal and respiratory system; age-related physical and mental deterioration (e.g., Alzheimer's Disease and age-related dementia); cardiovascular disease; cerebral vascular insufficiency and impaired cerebral performance; congestive symptoms of premenstrual syndrome; allergies; age-related vision loss; depression; Raynaud's disease; peripheral vascular disease; intermittent claudication; vertigo; equilibrium disorder; prevention of altitude sickness; tinnitus (ringing in the ear); liver fibrosis; macular degeneration; asthma; graft rejection; and immune disorders that induce toxic shock; bronchopulmonary disease as cystic fibrosis; chronic bronchitis; gastritis; heart attack; angina pectoris; chronic obstructive pulmonary disease; kidney damage during coronary angiography; Unverricht-Lundborg disease; pseudo porphyria; pneumonia; and paracetamol hepatotoxicity

In particular embodiments, the disease is a cancer.

One definition of cancer relates a disease characterized by the development of abnormal cells that divide uncontrollably and have the ability to infiltrate and destroy normal body tissue.

As the composition or kit of the invention is a selective a potent regulator of cell growth as disclosed here, its efficiency applies to cancer. In particular embodiments, a cancer is selected from the group consisting of breast cancer, prostate cancer, liver cancer, pancreas cancer, lung cancer, airway, upper and/or lower digestive tract cancer, organs of digestion cancer, kidney cancer, urinary tract cancer, genital organs cancer, skin cancer, ENT sphere cancer, and lymphatic organs cancers, liquid cancers.

The composition or kit of the invention is provided for use in preventing and/or treating a pancreatic cancer, more particularly a metastatic pancreatic cancer.

The composition or kit of the invention is provided for its use in preventing and/or treating a pancreatic cancer as an adjuvant or neoadjuvant to anti-pancreatic cancer treatment.

The composition or kit of the invention for use in preventing and/or treating a liver cancer, more particularly a metastatic liver cancer.

The composition of the invention for its use in preventing and/or treating liver cancer as an adjuvant or neoadjuvant to liver cancer treatment.

Any of the composition above can be given in combination with another drug.

Most strikingly, the applicant identified a potent activity of said composition per se, in the preventing and/or treating a cancer, in particular a metastatic cancer, and more particularly a metastatic cancer of the liver or of the pancreas.

The present invention relates to a composition or kit according to the above embodiments used as a drug acting alone to treat a disease, or a complication (e.g. angiogenesis, metastasis dissemination) of the disease, or at an advanced stage of the disease.

The present invention relates to the anti-cancer and/or anti-metastatic properties of the composition of the invention which is used as a drug in the preventive and/or curative treatment of cancer, in particular liver cancer or cancer of the pancreas, more particularly metastatic form of hepatic cancer or of pancreatic cancer.

While studying the activity of the composition, the applicant discovered that the new composition of the invention also exhibits a potent activity in preventing or inhibiting side effects observed in patients treated with drugs, in particular those related to an anti-cancer drug, such as an anti-metabolite or an anti-folic drug, exemplified as 5-FU or a precursor of 5-FU, such as capecitabine.

Remarkably, the composition of the invention when administered to individuals suffering side effects of a drug was found to reduce very significantly the side effects due to the drug.

Thus, is provided a composition or kit according to the present invention for use in reducing a side effect due to a drug, in particular due to any drug cited here.

A side effect that can be attenuated thanks to the composition or the kit of the invention can be any side effect due to a medicament. Thus, the composition or kit according to any one of the compositions or kits described here is provided for use in the prevention and/or the treatment of a side effect induced by a drug, preferably a side effect selected from the list consisting of weight loss, nausea, vomiting, headaches, bleeding, hair loss, skin rash, ocular irritation, neutropenia, pain, blood clots, lymphedema, a combination of said side effects, advantageously weight loss.

### EXAMPLES

Example 1. Effect of a composition comprising FAD, ATP, GSH and optionally myristic acid in a model of aggressive pancreatic cancer in vivo.

### Methods

Reagents: the FAD molecule was obtained from Alfa Aesar or abcr.

PEG was purchased at Sigma Aldricht under ref 8.43912.

Myristic acid was purchased at Sigma Aldricht under ref 8.00399.

Glutathione was purchased at Sigma Aldricht under ref G4251.

ATP was purchased at Sigma Aldricht under ref A26209.

FAD - polymer and FAD - polymer -metal salt were prepared as previously disclosed in WO2021084166A1 which is herewith enclosed in its entirety and a composition A and a composition B were prepared by completing FAD - polymer and FAD - polymer -metal salt with ATP, then GSH and myristic acid (for composition A).

Each mouse was subcutaneously treated with Pan02 cell suspension (5 * 10⁵ cells) in the right axilla of the forelimb. When the tumor size reaches 100 mm³-150 mm³, mice were randomly divided into the following treatment groups (n=10): the model group (n=10), the A group (n=10), the B group (n=10), the 5-FU group (1.5 mg/kg, n=10), the A+5-FU group (1.5 mg/kg, n=10) or A+5-FU (0.75mg/kg), the B+5-FU group (1.5 mg/kg, n=10) (Table 1). 100 µl of the different compositions were administered either intravenously (i.v.) or/and intraperitoneally (i.p.) once every other day (see Table 1 below). Body weight was weighed every three days. The volume of the solid tumor was measured using a digital caliper every three days. The survival of animals was monitored and recorded daily.

### Results

Compared with the model group, neither A, nor B modified the body weight changes of Pan02 tumor-bearing mice. The body weight of the mice in the positive control drug 5-FU group (1.5 mg/kg) decreased with time. Noticeably, the body weight of the mice in the A+5-FU (1.5 mg/kg) group did not decrease at all.

On the 82nd day, the mortality rate of mice in each group was: for model group, 90% and 100% (in two different independant experiments); A group, 70%; B group, 90%; 5-FU (1.5 mg/kg) group, 90%; A+5-FU (1.5 mg/kg) group, 70%; A+5-FU (0.75 mg/kg) group, 90%; B+5-FU (1.5 mg/kg), 90%.

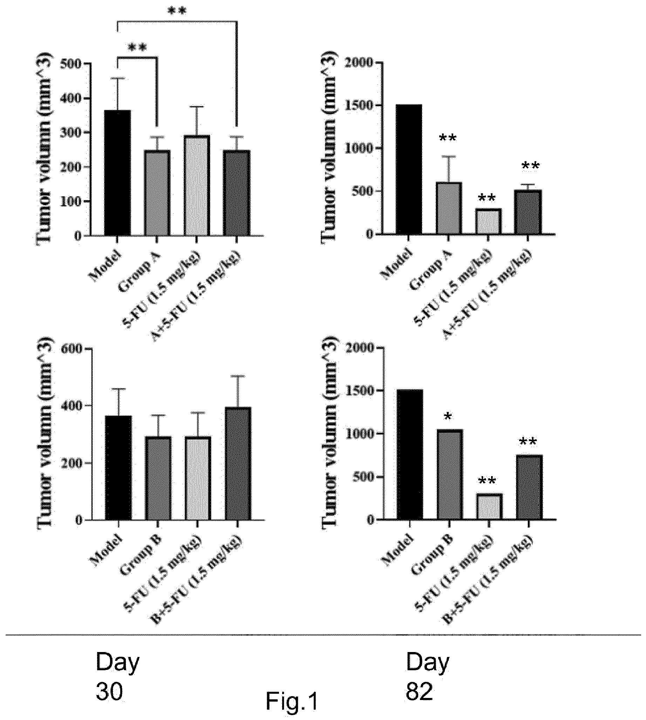

The results from tumor volume growth curves indicate that tumor volume of the mice in model group increased rapidly. In contrast, the A+5-FU (1.5 mg/kg) group decreased significantly the tumor volume, and most significantly as compared to any other group.(Fig. 1).

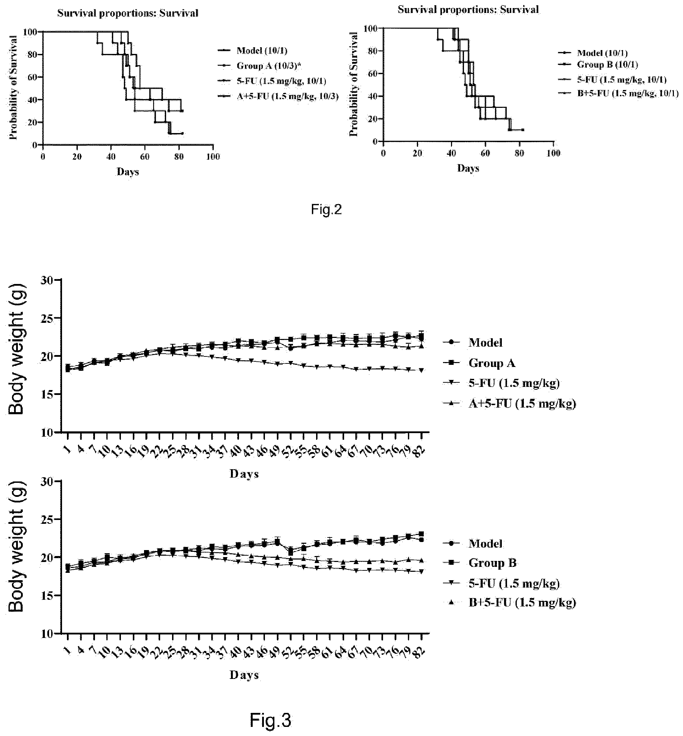

The results Fig 2 related to mortality indicate that all of the mice in model group died within 82 days owing to the significant fast growth of Pan02 transplanted pancreatic tumor. The median survival times of mice in each group are as follows: Model group, 48.5 days; material A group, 58.5 days; material B group, 51.5 days; 5-FU (1.5 mg/kg) group , 53.5 days; A+5-FU (1.5 mg/kg) group, 63.5 days; A+5-FU (0.75 mg/kg) group, 59.5 days; B+5-FU (1.5 mg/kg) group, 53.5 days. These findings clearly demonstrate that A + 5-FU group treatment greatly prolonged the survival period of Pan02 tumor bearing mice as compared to model/control group.

### Summary

Both composition A and composition B can reduce tumor size, and composition I A is better than composition B at reducing tumor size. The antitumor effect of composition A is equivalent to slightly better than that of 5-FU (1.5 mg/kg) group. The anti-tumor effect and prolong the survival of mice with pancreatic cancer in mce treated with composition A combined with 5-FU (1.5 mg/kg) group were better than the same effect measured in composition A- or 5-FU group alone- treated mice.

### 2. Compliance

This study was performed under the non-GLP specification and the mice were used in this study and fed in an animal room of SPF grade.

### 3. Abbreviations

| | | | |
|---|---|---|---|
| w | week | h | hour |
| °C | Celsius | mL | milliliter |
| g | gram | mg | milligram |
| ng | nanogram | s | second |
| kg | Kilogram | d | day |
| mmol | millimole | L | litre |
| min | minute | µL | microlitre |
| µm | micron | i.p. | intraperitoneally |
| i.v | intravenous | Mean | average value |
| SD | standard deviation | | |

### 4. Objective

To explore the effect of compositions A and B on life extension in Pan02 tumor-bearing mice.

### 5. Materials and instruments

### 5.1. Materials

FAD was purchased at abcr ref AB252055 - and made at a dose of 5mg/ml.

PEG was purchased at Sigma Aldricht under ref 8.43912 - 500µl for 20 ml of FAD at 5 mg/ml. myristic acid was purchased at Sigma Aldricht under ref 8.00399 used at 2 mg/ml. Glutathione was purchased at Sigma Aldricht under ref G4251 used at 5 mg/ml. all are dissolved or suspended in deionized water.

ATP was purchased at Sigma Aldricht under ref A26209 at 5mg/ml.

Test article: FAD/PEG600 complex as prepared in WO2020089310A1.
Purity: -.at least 98%
Appearance: liquid.
Storage: Stored at 4 ºC, away from light.
Registration procedure: The quantity, date, user, and purpose of use of the test sample are registered every time;

The solution has been prepared in advance according to the dose to be administered and stored at 4 °C in the dark.

20 ml of FAD (100 mg in 20 mL deionized water) were mixed with 500 µl PEG600 and stirred for 60min at room temperature (18-25°C).

After this time 5mg/ml of GSH was added allowed to age for 10 min, then 5 mg/ml of ATP optionally followed by 2mg/ml myristic acid (for composition A only). The resulting solution was sonicated for 2 hours and purified by dialysis through a membrane dialysis tubing Spectra /pore 3 (molecular weight cut off 1500 Da Serva electrophoresis, Germany).

Composition A: FAD (Flavin adenine dinucleotide disodium salt hydrate) + (PEG) Polyethylene glycol diacid 600 + myristic acid + L-Glutathione reduced (GSH) + ATP (Adenosine 5'Triphosphate disodium salt hydrate).

Composition B : FAD (Flavin adenine dinucleotide disodium salt hydrate)) + (PEG) polyethylene glycol diacid 600 + L-Glutathione reduced (GSH) + ATP (Adenosine 5'Triphosphate disodium salt hydrate). Positive control drug: 5-fluorouracil (5-FU), Lot number: H31020593. Manufacturer: Shanghai Xudong Haipu Pharmaceutical *Co., Ltd*.Storage: Sealed and stored at a temperature below 4 ºC.

Registration procedure: The number, date, user, and purpose of each test-sample are registered. Cultrex^{®}Basement membrane matrix HC - Manufacturer: Corning, USA.

DMEM: Catalog: 11965092; Manufacturer: Gibco, USA.

Fetal Bovine Serum, qualified, Australia- Catalog: 10099141C; Manufacturer: Gibco, USA. Trypsin-EDTA (0.05%), phenol red- Catalog: 25300054;- Manufacturer: Gibco, USA.

HBSS, no calcium, no magnesium, no phenol red- Catalog: 14175095; Manufacturer: Sigma-Aldrich, USA.

PBS powder- Manufacturer: Sigma-Aldrich, USA.

Paraformaldehyde- Catalog: 20100601-1; - Manufacturer: Guangzhou Chemical Reagent Factory. Isoflurane- Validity period: 2019-12-28; Manufacturer: Shenzhen Ruiwode Life Technology *Co., Ltd.*

### 5.2. Instruments

Animal Weighing Balance- Catalog: JCS-11001C; Manufacturer: Shanghai Ranhao Electronics *Co., Ltd.* Drug Weighing Balance- Catalog: BSM-1204; Manufacturer: Shanghai Zhuojing Electronic Technology *Co., Ltd.*

Zinc alloy number display caliper- Catalog: 20050430; Manufacturer: Shenzhen Cebao Electronic Tools Firm.

Variable Speed Multipurpose Oscillator. Catalog: HY-4A; Manufacturer: Hunan Frontier Technology *Co*., *Ltd.*

Inverted fluorescence microscope- Catalog: Olympus IX71; Manufacturer: Olympus Corporation.

CO₂ incubator- Manufacturer: Thermo Fisher Scientific Inc. Low-temperature High-speed Centrifuge

Catalog: 5430/5430R; Manufacturer: Eppendorf Corporation.- Milli-Q^{®} Direct Water Purification System

Catalog: Advantage A10;- Manufacturer: Millipore Corporation, Merck KGaA, Darmstadt, Germany.

### 5.3. Animals

Species: Rodent;- Strain: C57BL/6j mice;- Grade: SPF; Gender: Female; Age: 6-8 weeks.

Animals were randomly divided into. groups of 10 mice and treated as in the Table 1 below: the model group (n=10), the A group (n=10), the B group (n=10), the 5-FU group (1.5 mg/kg, n=10), the A+5-FU group (1.5 mg/kg, n=10), the B+5-FU group (1.5 mg/kg, n=10).

Animal production license number: SCXK (Yue) 2021-0059, Guangzhou Ruige Biotechnology *Co., Ltd.;* Animal certificate number: No.44827200002020; No.44827200002058; Animal Use License number: SYXK(Yue)2021-0259, Guangzhou Ruige Biotechnology *Co., Ltd.;* Receiving date: Aug 5 and 7, 2022;

### 6. Experimental methods

### 6.1. Pan02 cell culture

The Pan02 mouse pancreatic cancer line was purchased from Shanghai Enzyme Research Biotechnology *Co., Ltd.* (Shanghai, China, Cell line No.: CC-Y2144) and stored at -80 °C after arrival. The Pan02 cells were cultured in DMEM supplemented with 10% FBS (v/v), 100 U/mL penicillin G (Sigma-Aldrich, USA), and 100 µg/mL streptomycin (Sigma-Aldrich, USA). The cultures were maintained in a humidified incubator at 37°C and 5% CO₂.

### 6.2. Quarantine

After entering the SPF-level experimental center, the nude mice were quarantined in strict accordance with the relevant technical requirements of the SPF-level experimental animal center of Guangzhou Ruige Biotechnology *Co., Ltd.* The quarantine time was 3 to 5 days. The general appearance and exercise conditions including state of consciousness, gait, response to stimulation, walking balance and limb coordination in rat were observed and recorded.

### 6.3. Grouping and administration

**Table 1 : Experimental animals were randomly divided into groups:**

| Group | Dose | Number of mice | Method of Administratio n | Administratio n Frequency |
|---|---|---|---|---|
| Model | Saline (100 µL) | 10 | i.v. | Once every other day |
| A | A (100 µL, corresponding to bottle bottom No. 1) | 10 | i.v. | Once every other day |
| B | B (100 µL, corresponding to bottle bottom No. 2) | 10 | i.v. | Once every other day |
| 5-FU | 5-FU (1.5 mg/kg) | 10 | i.p. | Once every other day |
| A+5-FU | A (100 µL) + 5-FU (1.5 mg/kg) | 10 | i.v. + i.p. | Once every other day |
| B+5-FU | B (100 µL) + 5-FU (1.5 mg/kg) | 10 | i.v. + i.p. | Once every other day |

Different test drugs were administered intravenously (i.v.) and intraperitoneally (i.p.) once every other day at 10:00 am. A or B drugs were injected into the tail vein first, and 5-FU was injected intraperitoneally 24 h later.

### 6.4. Model establishment, grouping, and administration methods

Each mouse was subcutaneously treated with 0.1 mL cell suspension (5 * 10⁵ cells) in the right axilla of the forelimb. When the tumor size increases to 100 mm³-150 mm³, mice were randomly divided into treatment groups: the model group (n=10), the A group (n=10), the B group (n=10), the 5-FU group (1.5 mg/kg, n=10), the A+5-FU group (1.5 mg/kg, n=10), the B+5-FU group (1.5 mg/kg, n=10) (Table 1).

### 6.5. Detection Indicators

Body weight was weighed every three days. The length and width of the solid tumor was measured with a digital caliper every three days. Tumor volume was calculated by the modified ellipsoidal formula: V = π/6 × (length × width²). The survival time of animals was monitored and recorded daily. The test continued for 82 days and those that lived more than 82 days were defaulted as 82 days. The percent survival (%) was calculated using the following equations: percent survival (%) = [(10 - numbers of mice died in each group)/10] * 100%. Cytokine /numeration was measured in blood.

### 6.6. Statistical analysis

The GraphPad Prism 9.0 software (San Diego, CA, USA) was used to analyze the data. Data were expressed as mean ± SD. One-way ANOVA, two-way ANOVA and Tukey's test were used for comparison between groups. A value of p < 0.05 was considered statistically significant.

### 7. Results

### 7.1 A and B reverse the 5-FU-induced body weight loss

As shown in Fig. 3, compared with the model group, A and B did not affect the body weight of Pan02 tumor-bearing mice. As expected, the body weight of the mice in the positive control drug 5-FU group (1.5 mg/kg) decreased during the administration. Surprisingly, the body weight of the mice in the A+5-FU (1.5 mg/kg) group or in the B group, did not decrease.

Fig..3. Effect of A (right histogram) or B (left histogram) on 5-FU-induced body weight decrease in Pan 02 tumor-bearing mice. Body weight was shown as Mean ± SD. All data were analyzed using two-way ANOVA, followed by Tukey's multiple comparisons test.

### 7.2 A reduced the mortality of Pan02 tumor-bearing mice

As shown in Fig. 2, on the 82nd day, the mortality rate of mice in each group was: model group, 90% or 100% (in two different expriments); A group, 70%; B group, 90%; 5-FU (1.5 mg/kg ) group 90%; A+5-FU (1.5 mg/kg) group, 70%; B+5-FU (1.5 mg/kg), 90%. A reduced the mortality of Pan02 tumor-bearing mice.

### 2. Effect of A and B on mortality rate in Pan 02 tumor-bearing mice.

### 7.3 Effects of A and B on Pan02 tumor growth

The antitumor effect of A and B on Pan02 tumor-bearing mice is summarized in Fig.1 . As shown in Fig. 1, the results from tumor volume growth curves clearly indicate that tumor volumes of the mice in model groups and in B treatment increased rapidly during the 82 days duration. Noticeably, 5-FU and A significantly decreased tumor volume, and A to the same extent than 5-FU, the A+5-FU (1.5 mg/kg) group decreased the tumor volume most significantly.

Effect of A and B treatment reduced tumor growth *in vivo.* The longest diameter and perpendicular short diameter of the tumor were recorded every 3 days. Tumor volume was shown as Mean ± SD.

### 7.4 Effect of A and B on life extension of mice

Evaluation of the effect of A and B on life extension in Pan02 tumor-bearing mice was accomplished and illustrated in Fig. 2. The results indicate that all of the mice in model group died within 82 days owing to the significant fast growth of Pan02 transplanted tumor. The median survival times of mice in each group are as follows: Model group, 48.5 and 49.5 days; material A group, 58.5 days; material B group, 51.5 days; 5-FU (1.5 mg/kg) group, 53.5 days; A+5-FU (1.5 mg/kg) group, 63.5 days; A+5-FU (0.75 mg/kg) group, 59.5 days; B+5-FU (1.5 mg/kg) group, 53.5 days. These findings clearly demonstrate that A + 5-FU group treatment greatly and significantly prolonged the survival period of Pan02 tumor bearing mice.

Fig.2. A and B administration prolonged the survival of Pan 02 tumor-bearing mice. Log-rank (Mantel-Cox) test (recommended) analysis was used. *P<0.05 vs Model group, #P<0.05 vs 5-FU (1.5 mg/kg) group.

### 8. Conclusion

Both material A and material B can reduce pancreatic tumor size, and material A is better than material B. The antitumor effect of material A is equivalent to that of 5-FU (1.5 mg/kg) group. The anti-tumor effect and prolong the survival of mice with pancreatic cancer in material A combined with 5-FU ( 1.5 mg/kg) group were better than material A or 5-FU group alone.

### Example 2. Evaluation of the effect of composition A and B on life extension in H22 tumor (aggressive liver cancer)-bearing mice

### Methods

Each mouse was subcutaneously treated with H22 cell suspension (2 * 10⁶ cells) in the right axilla of the forelimb. When the tumor size increases to 100 mm³-150 mm³, mice were randomly divided into eight treatment groups: the model group (n=10), the A group (n=10), the B group (n=10), the 5-FU group (2.5 mg/kg, n=10), the A+5-FU group (1.5 mg/kg, n=10), the B+5-FU group (1.5 mg/kg, n=10). Different test drugs were administered intravenously (i.v.) or/and intraperitoneally (i.p.) once every two days. Body weight was weighed every three days. The volume of the solid tumor was measured with a digital caliper every three days. The survival time of animals was monitored and recorded daily.

### Results

Compared with the model group, A and B did not affect the body weight of H22 tumor-bearing mice. However, the body weight of the mice in the positive control drug 5-FU group (2.5 mg/kg) decreased during the experimentation. On the 22nd day, the mortality rate of mice in each group was: model group, 100%; A group, 50%; B group, 60%; 5-FU (2.5 mg/kg) group, 30%; A+5-FU (1.5 mg/kg) group, 30%; B+5-FU (1.5 mg/kg), 40%. The mortality rate of A+5-FU (1.5 mg/kg) group was equal to that of 5-FU (2.5 mg/kg) group. This shows a fast progression of cancer as compared even with pancreatic cancer used in the first experiments.

The results from tumor volume growth curves clearly indicate that tumor volumes of the mice in model group increased during the 22 days duration. In contrast, the treatment of A + 5-FU significantly attenuates the tumor growth. From the 13th day, the average tumor volume of the A combined 5-FU-treated mice increased relatively slowly. The results indicate that all of the mice in model group died within 22 days owing to the significant fast growth of H22 transplanted tumor. The median survival times of mice in each group are as follows: Model group, 15.5 days; material A group, 20 days; material B group, 18 days; 5-FU (2.5 mg/kg) group, 22 days; A+5-FU (1.5 mg/kg) group, 22 days; B+5-FU (1.5 mg/kg) group, 22 days. These findings clearly demonstrate that A + 5-FU group treatment greatly prolonged the survival period of H22 tumor bearing mice.

### Conclusion

Both A and B can reduce tumor size, but A is more efficient than B. The antitumor effects of A and B are slight but significant. The anti-tumor effect and prolonging the survival period of H22 tumor-bearing mice treated with A + 5-FU (1.5 mg/kg) group is equivalent to that of 5-FU (2.5 mg/kg) group.

### 5. Materials and instruments

### 5.1. Materials

### Test article: FAD and FAD/PEG complex

Storage: Stored at 4 ºC, away from light.

Registration procedure: The quantity, date, user, and purpose of use of the test sample are registered every time; The solution has been prepared in advance according to the dose to be administered and stored at 4 ºC in the dark.

Positive control drug: 5-fluorouracil injection- Lot number: H31020593.- Manufacturer: Shanghai Xudong Haipu Pharmaceutical *Co., Ltd.* Storage: Sealed and stored at a temperature below 4 ºC.

Registration procedure: The number, date, user, and purpose of each test sample are registered. Cultrex^{®}Basement membrane matrix HC - Manufacturer: Corning, USA.

RPMI-1640- Catalog: 61870036; Manufacturer: Gibco, USA.

Fetal Bovine Serum, qualified, Australia- Catalog: 10099141C; Manufacturer: Gibco, USA. Trypsin-EDTA (0.05%), phenol red, Catalog: 25300054- Manufacturer: Gibco, USA.

HBSS, no calcium, no magnesium, no phenol red- Catalog: 14175095, Manufacturer: Sigma-Aldrich, USA

PBS powder- Manufacturer: Sigma-Aldrich, USA-

Paraformaldehyde- Catalog: 20100601-1; Manufacturer: Guangzhou Chemical Reagent Factory. Isoflurane- Validity period: 2019-12-28; Manufacturer: Shenzhen Ruiwode Life Technology *Co., Ltd.*

### 5.2. Instruments

Animal Weighing Balance- Catalog: JCS-11001C. Manufacturer: Shanghai Ranhao Electronics *Co., Ltd.* Drug Weighing Balance- Catalog: BSM-1204. Manufacturer: Shanghai Zhuojing Electronic Technology *Co., Ltd.*

Zinc alloy number display caliper- Catalog: 20050430; Manufacturer: Shenzhen Cebao Electronic Tools Firm.

Variable Speed Multipurpose Oscillator- Catalog: HY-4A; Manufacturer: Hunan Frontier Technology Co., *Ltd.*

Inverted fluorescence microscope- Catalog: Olympus IX71;- Manufacturer: Olympus Corporation. CO₂ incubator (Manufacturer: ThermoFisher Scientific Inc.- Low-temperature High-speed Centrifuge-Catalog: 5430/5430R; Manufacturer: Eppendorf Corporation.
and 100 µg/mL streptomycin (Sigma-Aldrich, USA). The cultures were maintained in a humidified incubator at 37 °C and 5% COz.

### 6.2. Quarantine

After entering the SPF-level experimental center, the nude mice were quarantined in strict accordance with the relevant technical requirements of the SPF-level experimental animal center of Guangzhou Ruige Biotechnology *Co., Ltd.* The quarantine time was 3 days. The general appearance and exercise conditions including state of consciousness, gait, response to stimulation, walking balance and limb coordination in rat were observed and recorded.

### 6.3. Grouping and Administration

**Table 2 : Experimental animals were randomly divided into 8 groups:**

| Group | Dose | Number of mice | Method of Administratio n | Administratio n Frequency |
|---|---|---|---|---|
| Model | Saline (100 µL) | 10 | i.v. | Once every two days |
| A | A (100 µL, corresponding to bottle bottom No. 1) | 10 | i.v. | Once every two days |
| B | B (100 µL, corresponding to bottle bottom No. 2) | 10 | i.v. | Once every two days |
| 5-FU | 5-FU (2.5 mg/kg) | 10 | i.p. | Once every two days |
| A+5-FU | A (100 µL) + 5-FU (1.5 | 10 | i.v. + i.p. | Once every |

Milli-Q^{®} Direct Water Purification System- Catalog: Advantage A10; Manufacturer: Millipore Corporation, Merck KGaA, Darmstadt, Germany.

### 5.3. Animals

Species: Rodent; Strain: BALB/c mice; Grade: SPF; Gender: Female; Age: 5-6 weeks. Animal production license number: SCXK (Yue) 2020-0051, Zhuhai Baishitong Biotechnology *Co., Ltd.;*
Animal certificate number: No.44822700008254; No.44822700008373;
Animal Use License number: SYXK(Yue)2021-0259, Guangzhou Ruige Biotechnology Co., Ltd.;

The animals were fed in an animal room of SPF grade of Guangzhou Ruige Biotechnology *Co., Ltd.* The mice had free access to food and water throughout the experiment and were housed under 12:12 h light/dark conditions in a temperature-controlled environment (20-26 °C) and the humidity being controlled at 40-70%. Experimental procedures were conducted and approved by the Experimental Animal Ethics Committee of Guangzhou Ruige Biotechnology *Co., Ltd.*

The number of mice: Animals were randomly divided into eight groups: the model group (n=10), the A group (n=10), the B group (n=10), the 5-FU group (2.5 mg/kg, n=10), the A+5-FU group (1.5 mg/kg, n=10), the B+5-FU group (1.5 mg/kg, n=10).

### 6. Experimental methods

### 6.1. H22 cell culture

The H22 mouse hepatoma cell line was purchased from Wuhan Procell Life Technology *Co., Ltd.* (Wuhan, China, Contract No.: XD-000-2022-03-30-0029) and stored at -80 °C after arrival. The H22 cells were cultured in RPMI-1640 supplemented with 10% FBS (v/v), 100 U/mL penicillin G (Sigma-Aldrich, USA),

| | | | | |
|---|---|---|---|---|
| | mg/kg) | | | two days |
| B+5-FU | B (100 µL) + 5-FU (1.5 mg/kg) | 10 | i.v. + i.p. | Once every two days |

Different test drugs were administered intravenously (i.v.) and intraperitoneally (i.p.) once every two days at 10:00 am (Table 2). A or B drugs were injected into the tail vein first, and 5-FU was injected intraperitoneally after 24 h.

### 6.4. Model establishment, grouping, and administration methods

The Balb/c mice were intraperitoneally treated with H22 cells (1*10⁷ cells/mL, 0.2 mL) under sterile conditions and were sacrificed after 7 days to collect ascites (the collected ascitic tumor fluid was thick and ivory in color). Ascitic tumor fluid was then diluted with matrigel to adjust the cell concentration to 2 * 10⁷ cells/mL. Each mouse was subcutaneously treated with 0.1 mL cell suspension in the right axilla of the forelimb. When the tumor size increases to 100 mm³-150 mm³, mice were randomly divided into treatment groups: the model group (n=10), the A group (n=10), the B group (n=10), the 5-FU group (n=10), the A+5-FU group (2.5 mg/kg, n=10), the B+5-FU group (n=10).

### 6.5. Detection Indicators

Body weight was weighed every three days. The length and width of the solid tumor was measured with a digital caliper every three days. Tumor volume was calculated by the modified ellipsoidal formula: V = n/6 × (length × width²). The survival time of animals was monitored and recorded daily. The test continued for 22 days and those that lived more than 22 days were defaulted as 22 days. The percent survival (%) was calculated using the following equations: percent survival (%) = [(10 - numbers of mice died in each group)/10] * 100%.

### 6.6. Statistical analysis

The GraphPad Prism 9.0 software (San Diego, CA, USA) was used to analyze the data. Data were expressed as mean ± SD. One-way ANOVA, two-way ANOVA and Tukey's test were used for comparison between groups. A value of p < 0.05 was considered statistically significant.

### 7. Results

### 7.1 A and B did not affect body weight changes due to chemotherapy but prevent the side effects due to 5-FU

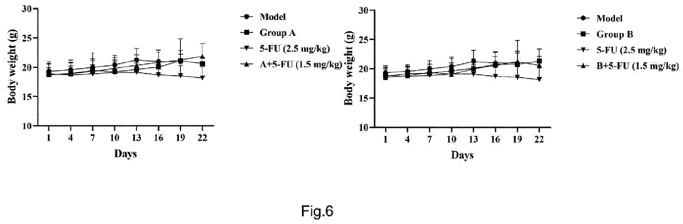

As shown in Fig. 6, compared with the model group, A and B did not affect the body weight of H22 tumor-bearing mice. However, the body weight of the mice in the positive control drug 5-FU group (2.5 mg/kg) decreased during the administration, but not if A or B is co- injected.

Fig.6. Effect of A and B on body weight changes in H22 tumor-bearing mice. Body weight was shown as Mean ± SD. All data were analyzed using two-way ANOVA, followed by Tukey's multiple comparisons test.

### 7.2 A combined with 5-FU reduces the mortalityof H22 tumor-bearing mice

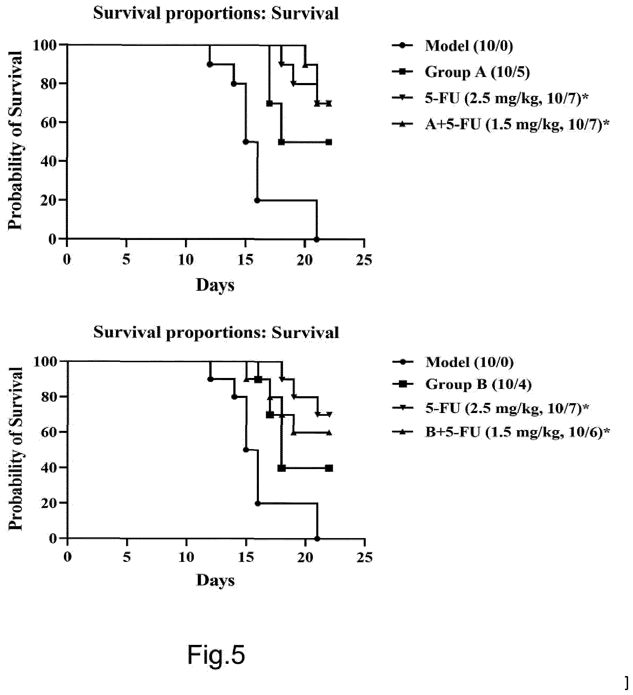

As shown in Fig.5, on the 22nd day, the mortality rate of mice in each group was: model group, 100%; A group, 50%; B group, 60%; 5-FU (2.5 mg/kg ) group, 30%; A+5-FU (1.5 mg/kg) group, 30%, 50%; B+5-FU (1.5 mg/kg), 40%. The mortality rate of A+5-FU (1.5 mg/kg) group was equal to that of 5-FU (2.5 mg/kg) group.

### 7.3 Effects of A and B on H22 tumor growth

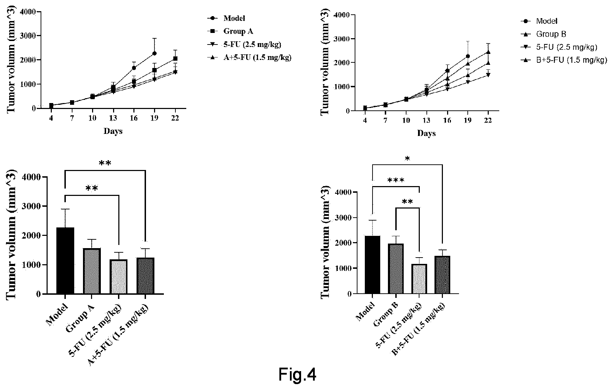

The antitumor effect of A and B on H22 tumor-bearing mice is summarized in Fig. 4. As shown in Fig. 4, the results from tumor volume growth curves clearly indicate that tumor volumes of the mice in model group increased rapidly during the 22 day duration. In contrast, the treatment of A + 5-FU suppressed the tumor growth. From the 13th day, the average tumor volume of the A combined 5-FU-treated mice increased relatively slowly.

### 7.4 Effect of composition A and composition B on life extension of mice suffering liver cancer

Evaluation of the effect of A and B on life extension in H22 tumor-bearing mice was accomplished and displayed in Fig.5. The results indicate that all of the mice in model group died within 21 days owing to the signifcant fast growth of H22 transplanted tumor. The median survival times of mice in each group are as follows: Model group, 15.5 days; material A group, 20 days; material B group, 18 days; 5-FU (2.5 mg/kg) group , 22 days; A+5-FU (1.5 mg/kg) group, 22 days, 21.5 days; B+5-FU (1.5 mg/kg) group, 22 days. These findings clearly demonstrate that A + 5-FU group treatment greatly prolonged the survival period of H22 tumor bearing mice;

### 8. Conclusion

Both A and B can reduce liver tumor size, but A is better than B. The antitumor effects of A and B is significant.

A and B abolish the side effects of 5-FU, in particular weight loss.

## Claims

1. A composition comprising Flavine adenine dinucleotide (FAD) or a salt thereof, reduced glutathione (GSH), adenosine triphosphate (ATP) or a salt thereof, optionally myristic acid, or a salt thereof, and a carrier.

2. The composition according to claim 1 wherein the salt of FAD is Flavin adenine dinucleotide disodium salt hydrate, the salt of ATP is Adenosine Triphosphate disodium salt hydrate, GSH is reduced L-Glutathione.

3. The composition according to claim 1 or 2, wherein the carrier, is chosen from a metal salt, a polymer, a liposome, an excipient, a combination thereof.

4. The composition according to any one of claim 1 to 3, wherein the carrier comprises a polymer selected from polyethylene glycol (PEG), chitosan, collagen, starch, hyaluronic acid preferably a PEG, or chitosan or hyaluronic acid more preferably PEG 600; even more preferably PEG-600-diacid and even more more preferably hyaluronic acid.

5. The composition according to any one of claim 1 to 4, wherein the carrier comprises a metal salt, preferably a gold salt, more preferably HAuCl4*6H20.

6. The composition according to anyone of the preceding claim 1 to 5 in a form suitable for intravenous administration (iv), oral including sublingual administration (po), rectal administration, intratumoral administration, intraocular administration, intramucosal administration, intra-vesical administration, or intra-urethral administration, advantageously iv or po administration.

7. The composition according to any one of claim 1 to 6 wherein the composition is administered to an individual at a dose ranging from 0,1mg to 10g for FAD or a salt thereof, a dose ranging from 0,1 to 10 g for GSH, a dose ranging from 0,1mg to 10g for ATP or a salt thereof and a dose ranging from 0.1mg to 10g for myristic acid or a salt thereof,

8. The composition according to any one of claims 1 to 7 in a therapeutically effective amount and a pharmaceutically acceptable carrier, or a nutraceutically acceptable carrier.

9. The composition according to any one of claims 1 to 8 and a medicament.

10. The composition according to any one of claims 1 to 9, for use in the prevention and/or the treatment of a disease.

11. The composition for its use according to claim 10 wherein the disease is a cancer.

12. The composition according to claim 11 for its use as an adjuvant (complement) or neoadjuvant (debulking) to cancer treatment.

13. The composition according to any one of claim 11 to 13 wherein the disease is selected from the group consisting of brain cancer, breast cancer, prostate cancer, lung cancer, airway cancer, upper and/or lower digestive tract cancer, stomach cancer, kidney cancer, pancreas cancer, liver cancer, urinary tract cancer, genital organs cancer, skin cancer, Ear, Nose and Throat (ENT) sphere cancer, and lymphatic organs cancer, preferably pancreas cancer or liver cancer, more preferably aggressive of metastatic tumor cancer.

14. The composition according to any one claim 1 to 13 for use in the prevention and/or the treatment of a side effect induced by a drug or a disease.

15. The composition according to any one claim 1 to 14 for use in the prevention and/or the treatment of a side effect induced by 5-FU.

16. The composition according to any one claim 1 to 15 for use in the prevention and/or the treatment of a side effect said side effect being selected from the list consisting of weight loss, nausea, vomiting, headache, bleeding, hair loss, neutropenia, pain, blood clots, lymphedema, a combination of said side effects, advantageously weight loss.
